# EUROPEAN PATENT SPECIFICATION

(11) **EP 2 328 402 B1**
(45) Date of publication and mention of the grant of the patent: **01.06.2016**
(21) Application number: 09810283.3
(22) Date of filing: 28.08.2009
(51) Int. Cl.: A01H 4/00, C12N 15/82

(54) **METHODS AND COMPOSITIONS FOR INCREASING STORAGE-LIFE OF FRUIT**
VERFAHREN UND ZUSAMMENSETZUNGEN ZUR VERLÄNGERUNG DER HALTBARKEIT EINER FRUCHT
PROCÉDÉS ET COMPOSITIONS POUR AUGMENTER LA DURÉE DE STOCKAGE DE FRUITS

(30) Priority: 29.08.2008 NZ 57088608
(43) Date of publication of application: 08.06.2011
(73) Proprietor: The New Zealand Institute for Plant and Food Research Limited, Mt Albert, Auckland (NZ)
(72) Inventor: ATKINSON, Ross Graham, Mt Albert, Auckland (NZ); SCHAFFER, Robert James, Mt Albert, Auckland (NZ); GUNASEELAN, Kularajathevan, Mt Albert, Auckland (NZ); SCHRÖDER, Roswitha, Henderson, Auckland (NZ)
(74) Representative: Kremer, Simon Mark
(86) International application number: PCT/NZ2009/000182
(87) International publication number: WO 2010/024699

(56) References cited:
- EP-A1- 1 270 729
- WO-A1-93/23551
- WO-A1-96/30529
- WO-A2-02/16613
- US-A- 4 801 540
- US-A1- 2004 250 322
- KRAMER, M., ET AL.: "Postharvest evaluation of transgenic tomatoes with reduced levels of polygalacturonase: processing, firmness and disease resistance", POSTHARVEST BIOLOGY AND TECHNOLOGY, vol. 1, no. 3, 1 March 1992 (1992-03-01), pages 241-255, XP002659871,
- PICTON S ET AL: "ALTERED FRUIT RIPENING AND LEAF SENESCENCE IN TOMATOES EXPRESSING AN ANTISENSE ETHYLENE-FORMING ENZYME TRANSGENE", PLANT JOURNAL, vol. 3, no. 3, 1 March 1993 (1993-03-01), pages 469-481, XP000673757, BLACKWELL SCIENTIFIC PUBLICATIONS, OXFORD, GB ISSN: 0960-7412
- SMITH C J S ET AL: "ANTISENSE RNA INHIBITION OF POLYGALACTURONASE GENE EXPRESSION IN TRANSGENIC TOMATOES", NATURE, vol. 334, 25 August 1988 (1988-08-25), pages 724-726, XP000196983, NATURE PUBLISHING GROUP, LONDON, GB ISSN: 0028-0836, DOI: 10.1038/334724A0
- TACKEN EMMA ET AL: "The Role of Ethylene and Cold Temperature in the Regulation of the Apple POLYGALACTURONASE1 Gene and Fruit Softening", PLANT PHYSIOLOGY (ROCKVILLE), vol. 153, no. 1, May 2010 (2010-05), pages 294-305, XP002659872, ISSN: 0032-0889
- COSTA FABRIZIO ET AL: "QTL dynamics for fruit firmness and softening around an ethylene-dependent polygalacturonase gene in apple (Malusxdomestica Borkh.)", JOURNAL OF EXPERIMENTAL BOTANY, vol. 61, no. 11, June 2010 (2010-06), pages 3029-3039, XP002659873, ISSN: 0022-0957
- QUESADA MIGUEL A ET AL: "Antisense Down-Regulation of the FaPG1 Gene Reveals an Unexpected Central Role for Polygalacturonase in Strawberry Fruit Softening", PLANT PHYSIOLOGY (ROCKVILLE), vol. 150, no. 2, June 2009 (2009-06), pages 1022-1032, XP002659874, ISSN: 0032-0889
- RONGCAI YUAN ET AL.: 'Effect of sprayable 1-MCP, AVG, and NAA on ethylene biosynthesis, preharvest fruit drop, fruit maturity and quality of 'Delicious' apples.' HORTSCIENCE. vol. 43, no. 5, 01 August 2008, pages 1454 - 60, XP008143275
- ATKINSON RG ET AL.: 'Overexpression of polygalacturonase in transgenic apple trees leads to a range of novel phenotypes involving changes in cell adhesion.' PLANT PHYSIOL. vol. 129, no. 1, May 2002, pages 122 - 33, XP008143250
- SEKINE D ET AL.: 'Cloning of cDNAs encoding cell-wall hydrolases from pear (Pyrus communis) fruit and their involvement in fruit softening and development of melting texture.' PHYSIOLOGIA PLANTARUM. vol. 126, no. 2, January 2006, pages 163 - 74, XP008144772
- ATKINSON RG ET AL.: 'Apple ACC-oxidase and polygalacturonase: ripening-specific gene expression and promoter analysis in transgenic tomato.' PLANT MOL BIOL. vol. 38, no. 3, October 1998, pages 449 - 60, XP008144773
- WAKASA Y ET AL.: 'Low expression of an endopolygalacturonase gene in apple fruit with long-term storage potential.' POSTHARVEST BIOLOGY AND TECHNOLOGY. vol. 39, no. 2, February 2006, pages 193 - 198, XP025139059
- ATKINSON RG.: 'A cDNA clone for endopolygalacturonase from apple.' PLANT PHYSIOL. vol. 105, no. 4, August 1994, pages 1437 - 8, XP008143252

### SEQUENCE LISTING

<110> THE NEW ZEALAND INSTITUTE FOR PLANT AND FOOD RESEARCH
   LIMITED
<120> METHODS AND COMPOSTIONS FOR INCREASING STORAGE-LIFE OF FRUIT
<130> 609739 HCF
<160> 7
<170> PatentIn version 3.5
<210> 1
   <211> 460
   <212> PRT
   <213> Malus x domestica
<400> 1
   Met Ala Leu Lys Thr Gln Leu Leu Trp Ser Phe Val Val Val Phe Val
   1               5                   10                  15
   Val Ser Phe Ser Thr Thr Ser Cys Ser Gly Ser Ser Phe Gln Glu Val
               20                  25                  30
   Asn Ala Leu His Ser Tyr Val Asp His Val Asp Asp Lys Glu Ser Gly
           35                  40                  45
   Tyr Asn Ser Arg Ala Tyr Pro Ser Tyr Thr Asp Thr Ile Glu Gly Leu
       50                  55                  60
   Lys Val Met Glu Leu Ile Arg Pro Arg Thr Gln Leu Phe Ser Ser Arg
   65                  70                  75                  80
   Lys Leu Asn Thr Ile Thr Gly Gly Ile Ala Thr Ser Ser Ala Pro Ala
                   85                  90                  95
   Lys Thr Ile Ser Val Asp Asp Phe Gly Ala Lys Gly Asn Gly Ala Asp
               100                 105                 110
   Asp Thr Gln Ala Phe Val Lys Ala Trp Lys Ala Ala Cys Ser Ser Ser
           115                 120                 125
   Gly Ala Met Val Leu Val Val Pro Gln Lys Asn Tyr Leu Val Arg Pro
       130                 135                 140
   Ile Glu Phe Ser Gly Pro Cys Lys Ser Gln Leu Thr Leu Gln Ile Tyr
   145                 150                 155                 160
   Gly Thr Ile Glu Ala Ser Glu Asp Arg Ser Ile Tyr Lys Asp Ile Asp
                   165                 170                 175
   His Trp Leu Ile Phe Asp Asn Val Gln Asn Leu Leu Val Val Gly Pro
               180                 185                 190
   Gly Thr Ile Asn Gly Asn Gly Asn Ile Trp Trp Lys Asn Ser Cys Lys
   195 200 205
   Ile Lys Pro Gln Pro Pro Cys Gly Thr Tyr Ala Pro Thr Ala Val Thr
   210 215 220
   Phe Asn Arg Cys Asn Asn Leu Val Val Lys Asn Leu Asn Ile Gln Asp
   225 230 235 240
   Ala Gln Gln Ile His Val Ile Phe Gln Asn Cys Ile Asn Val Gln Ala
   245 250 255
   Ser Cys Leu Thr Val Thr Ala Pro Glu Asp Ser Pro Asn Thr Asp Gly
   260 265 270
   Ile His Val Thr Asn Thr Gln Asn Ile Thr Ile Ser Ser Ser Val Ile
   275 280 285
   Gly Thr Gly Asp Asp Cys Ile Ser Ile Val Ser Gly Ser Gln Arg Val
   290 295 300
   Gln Ala Thr Asp Ile Thr Cys Gly Pro Gly His Gly Ile Ser Ile Gly
   305 310 315 320
   Ser Leu Gly Glu Asp Gly Ser Glu Asp His Val Ser Gly Val Phe Val
   325 330 335
   Asn Gly Ala Lys Leu Ser Gly Thr Ser Asn Gly Leu Arg Ile Lys Thr
   340 345 350
   Trp Lys Gly Gly Ser Gly Ser Ala Thr Asn Ile Val Phe Gln Asn Val
   355 360 365
   Gln Met Asn Asp Val Thr Asn Pro Ile Ile Ile Asp Gln Asn Tyr Cys
   370 375 380
   Asp His Lys Thr Lys Asp Cys Lys Gln Gln Lys Ser Ala Val Gln Val
   385 390 395 400
   Lys Asn Val Leu Tyr Gln Asn Ile Arg Gly Thr Ser Ala Ser Gly Asp
   405 410 415
   Ala Ile Thr Leu Asn Cys Ser Gln Ser Val Pro Cys Gln Gly Ile Val
   420 425 430
   Leu Gln Ser Val Gln Leu Gln Asn Gly Arg Ala Glu Cys Asn Asn Val
   435 440 445
   Gln Pro Ala Tyr Lys Gly Val Val Ser Pro Arg Cys
   450 455 460
<210> 2
   <211> 247
   <212> PRT
   <213> Malus *sieboldii*
<400> 2
   Met Ala Leu Lys Thr Gln Leu Leu Trp Ser Phe Val Val Val Phe Val
   1               5                   10                  15
   Val Ser Phe Ser Thr Thr Ser Cys Ser Gly Ser Ser Phe Gln Glu Val
               20                  25                  30
   Asn Ala Leu His Ser Tyr Val Asp His Val Asp Asp Lys Glu Ser Gly
           35                  40                  45
   Cys Asn Ser Arg Thr Tyr Pro Ser Tyr Thr Asp Thr Ile Ile Glu Gly
       50                  55                  60
   Leu Lys Phe Met Glu Leu Ile Arg Pro Ile Thr Gln Leu Phe Ser Ser
   65                  70                  75                  80
   Arg Lys His Asn Thr Ile Thr Gly Gly Ile Ala Thr Ser Ser Ala Arg
                   85                  90                  95
   Ala Lys Thr Ile Ser Val Asp Asp Phe Gly Ala Lys Gly Asp Gly Ala
               100                 105                 110
   Asp Asp Thr Gln Ala Phe Glu Lys Ala Trp Lys Ala Ala Cys Ser Ser
           115                 120                 125
   Ser Gly Ala Met Val Leu Val Val Pro Gln Lys Asn Tyr Leu Val Gly
       130                 135                 140
   Pro Ile Glu Phe Ser Gly Pro Cys Lys Ser Arg Leu Thr Leu Gln Ile
   145                 150                 155                 160
   Tyr Gly Thr Ile Glu Ala Ser Glu Asp Arg Ser Ile Tyr Lys Asp Thr
                   165                 170                 175
   Asp His Trp Leu Ile Phe Asp Asn Val Gln Asn Leu Val Val Val Gly
               180                 185                 190
   Pro Gly Thr Ile Asn Gly Asn Gly Asn Ile Trp Trp Lys Asn Ser Cys
           195                 200                 205
   Lys Ile Lys Pro Gln Pro Pro Cys Asp Thr Tyr Ala Pro Met Ala Val
       210                 215                 220
   Thr Phe Asp Lys Cys Asn Asn Leu Val Val Lys Asn Leu Asn Ile Arg
   225                 230                 235                 240
   Asp Ala Gln Gln Met His Val
                       245
<210> 3
   <211> 187
   <212> PRT
   <213> Malus *sieboldii*
<400> 3
   His Val Ser Asn Thr Gln Asn Ile Thr Ile Ser Ser Ser Val Ile Gly
   1               5                   10                  15
   Thr Gly Asp Asp Cys Ile Ser Ile Val Ser Gly Ser Gln Arg Val Gln
               20                  25                  30
   Ala Thr Asp Ile Thr Cys Gly Pro Gly His Gly Ile Ser Ile Gly Ser
           35                  40                  45
   Leu Gly Glu Asp Gly Ser Lys Asp His Val Ser Gly Val Phe Val Asn
       50                  55                  60
   Gly Ala Lys Leu Ser Gly Thr Ser Asn Gly Leu Arg Ile Lys Thr Trp
   65                  70                  75                  80
   Glu Gly Gly Ser Gly Ser Ala Thr Asn Ile Val Phe Gln Asn Val Gln
                   85                  90                  95
   Met Asn Asp Val Ala Asn Pro Ile Ile Ile Asp Gln Asn Tyr Arg Asp
               100                 105                 110
   His Lys Thr Lys Asp Cys Lys Gln Gln Lys Ser Ala Val Gln Val Lys
           115                 120                 125
   Asn Val Leu Tyr Lys Asn Ile Arg Gly Thr Ser Ala Ser Arg Asp Ala
       130                 135                 140
   Ile Thr Leu Asn Cys Ser Pro Ser Val Pro Cys Gln Gly Ile Val Leu
   145                 150                 155                 160
   Gln Ser Val Gln Leu Gln Asn Gly Arg Ala Glu Cys Asn Asn Val Lys
                   165                 170                 175
   Pro Ala Tyr Lys Gly Val Val Ser Pro Arg Cys
               180                 185
<210> 4
   <211> 2839
   <212> DNA
   <213> Malus x domestica
<400> 4
   gaattcaaaa aaactatttg gaccattccg agcaagtcta tctaaatagc aatagcatca      60
   ctctatatta ttgcagttct tattaagagt gtgtataata ttgtacttgt atccaaaaca     120
   acaatcctgg ctatttttac atattcaata taaagttcca atacgatcac tgattgatta 180
   attggtatca agtacaatac ttctgcatct ctcttaaaaa acatcaacaa tgcattacgt 240
   gttgtagatt gttatcagag tcggccctgg cccagtgtca ccagggcgac cgcttgaggt 300
   ccaaaaaaac gagtggcacc aaaaaaaaat taagttggtt catatatgta taaggttttg 360
   ttttattctt gatatatagt aaacgctcat attaaatagc aatatgtttt agatgacagg 420
   gtggttttat ttggtttttc cgtgcccacc taggtttaat tccctctctc aacataaagg 480
   ggtagtttcc cttctatttt attcatgtct actttaaata acaaacaaac attaattagc 540
   ttattaaatg tggcattttc agtagtcgtt ttttgttttc agtttgaatt aactcgcaaa 600
   atcatgttca agtttgtcta aatataaatt taggtttttt atatttgttg ataatctttt 660
   catggttaag taataaactt gtgatcatta tctttttatt gaacgaagta ttgttgacaa 720
   tccaaaaata tcatcctaca cttctttgta taattttttc cttatgattt tatatatttg 780
   gaatgaaccg caacttttca tgagcgctcg aaaaacaaaa acatagttga attactatgt 840
   gccctttggt agtagcaatt tgttgttttc atttgttgct tacatataga aattagagat 900
   cttaaatatg aaaaattcat ttcaaaagtt tcataatgtg caagtagctt gtagatcagt 960
   tagttaaaag tgttcagctt gtcactcaat gactcgtttt caaatttcct caccgtattt 1020
   ctgatgagtt tagtgtaaat taccctatca tttgtcaaaa aagtcataat gtgaaaaagg 1080
   tctattttct ttaatattca atgtacaaca tacaaatact aagataaata attattttat 1140
   gtgaagttat gttagggcat atttttcaat gtcgcctagg gcctcataaa actcaggata 1200
   ggccttgatt gttatatatg gtactaaaca aaagtttcca aaatacaaag tttaaaaaga 1260
   ttcaagtgga attttgaaga attttaaaag tattataaat tttgataacc cctcgagttg 1320
   attaattgcg ggggatgtgg tcatcatgac cgctatggta gttcatggag caacctactt 1380
   gttcccccac ttatgattga cgcattttta ctgtatgaac atcataatca gaaccgttcg 1440
   ttctctttgt catcatcgaa agatcatata tgcaaaaact cattaaattg agagattttt 1500
   ttagccattc atatgtatca tacaaataga cggttcatca taaggatgct acttgttacc 1560
   aaaaacattg attggtttga catatatgga tgggtaaaca atctccaaat ggaatatttt 1620
   ttttagatat gatatttcga tgatgataaa gagaatgaac gggtcaaata ataatgttca 1680
   tatggtgaag atgcgtaaat cataagtgaa gggacaagtt ggttccctat ggaggacaca 1740
   agcattattc gtttttaggt gtattcaatt agaattgtaa atgaatctat aaaagttcag 1800
   gaatattcag tcagaatgtt aaacaagctt atagaactcc atacaaattc aggtgtatta 1860
   atcaattaaa attttaaagg attttataaa agtcaacaga aatctgagtg tattcaaaga 1920
   agattttgaa aaagtctaag aaagttagag tgtattgatc agtaataatt tgattttaaa 1980
   gaattttaaa atgatacatt ttagtaaatt tgaaggaatt tcatagagta tttaaccctt 2040
   aataaatcat acttctgtaa agtccattaa aaaactccat caactttcat aaattgaaac 2100
   atttttaaat ccataaaagt tgaaccgaat ctattttcat atattatttt atacatgcaa 2160
   cgacttacgt tgtaacataa gggatgcaat gcatggcgca gaaagtcata gagtcggcaa     2220
   agacatcatt tcgtctgaat ctctcatgtc cgagaaccca tacctcaaga gcccaagacg     2280
   acacaataca caacaatcac cgtcaatacc cttctcttcc gctgcctata aataccaatg     2340
   gaaatcccac gacattctca ccaaatcatc atcacttgaa cacaccaatc cttacacttc     2400
   tagctacaat tctaagtttc cattttccaa catcccatca cattgttcaa aaatatcatc     2460
   agcctcgagt tagggtttat tatccttcgt gaccctcctt ttagtatttg gttctttttg     2520
   aaagacgatt tgttaggtgt ttctaggcct ctagccatct tgtatctcac caaaaaaaaa     2580
   tatattacca gctgtttaca ccaaattaaa tagtaaaaag aaagcatcaa tggctttaaa     2640
   aacacagttg ttgtggtcat ttgttgttgt ttttgttgtt tccttcagta caacttcatg     2700
   ttctggtagt agtttccagg aggtcaacgc gcttcatagt tacgttgacc atgttgatga     2760
   taaagagtcc ggctataatt ctagggctta tccttcatac acggacacca tagaaggttt     2820
   aaaggtcatg gaattgatc                                                  2839
<210> 5
   <211> 1773
   <212> DNA
   <213> Malus x domestica
<400> 5
   aaccttacac ttcttctaca attctaagtt tccattttcc aacatcccat cacattgttc     60
   aaaaatatca tcagcctcga gttagggttt attatccttc gtgaccctcc ttttagtatt    120
   tggttctttt tgaaagacga tttgttaggt gtttctaggc ctctagccat cttgtatctc    180
   accaaaaaaa aatatattac cagctgttta caccaaatta aatagtaaaa agaaagcatc    240
   aatggcttta aaaacacagt tgttgtggtc atttgttgtt gtttttgttg tttccttcag    300
   tacaacttca tgttctggta gtagtttcca ggaggtcaac gcgcttcata gttacgttga    360
   ccatgttgat gataaagagt ccggctataa ttctagggct tatccttcat acacggacac    420
   catagaaggt ttaaaggtca tggaattgat caggccaaga actcagctct tcagttcaag    480
   gaagctcaac acaatcaccg gtgggatagc aacatcatca gctccggcca aaaccattag    540
   cgtcgacgat tttggagcta aagggaatgg tgctgatgac acacaggcat ttgtgaaggc    600
   atggaaggca gcttgttctt ccagtggagc tatggttctt gtggtaccac agaagaacta    660
   tcttgttagg ccgattgaat tctcaggccc atgcaaatct caacttacac tgcagattta    720
   tggaaccata gaagcatcag aagaccgatc aatctacaaa gacatagacc actggctcat    780
   ctttgacaat gtccaaaact tgctagttgt tggtcctgga accatcaatg gcaatggaaa    840
   catctggtgg aaaaactcat gcaaaataaa acctcagccc ccttgcggta catacgcccc    900
   cacggctgtg accttcaaca ggtgcaataa cttggtggtg aagaatctga atatccaaga    960
   cgcacaacaa atccatgtca tattccaaaa ctgcatcaac gttcaagctt cctgtctcac   1020
   ggtaactgca ccagaggaca gccctaatac ggacggaatt catgtgacaa atacccagaa   1080
   catcactatc tcgagctcgg ttataggaac aggtgatgac tgtatttcta ttgtgagtgg   1140
   gtcccaaaga gttcaagcca cagacattac ttgtggacca ggccatggaa tcagtattgg   1200
   tagcttggga gaagacggct cagaagatca tgtttcagga gtatttgtga atggagctaa   1260
   gctttcagga acctccaatg gactccggat caagacgtgg aagggaggct caggcagtgc   1320
   aaccaacatt gttttccaga atgtgcaaat gaacgatgtc accaacccca tcatcatcga   1380
   ccagaactac tgtgaccaca aaaccaaaga ttgcaaacaa cagaaatcgg cggtccaagt   1440
   gaaaaatgtg ttgtaccaaa acataagagg aacgagtgct tccggcgacg cgataacgtt   1500
   gaactgcagc caaagtgttc cttgtcaggg gatcgtgctg caaagtgttc aactgcagaa   1560
   tggaagagct gaatgcaaca atgttcagcc tgcttacaaa ggagttgtct cccctagatg   1620
   ttaaaaccta gggttcataa ttatgggcat tgtgaaatag attatgcaat tcttgtacca   1680
   attagcacat aaataattgt ttgtttgtaa tatttatgtt taatttggca ttgtacataa   1740
   acatattcat aaataaaaag atgcaacttt tat                                1773
<210> 6
   <211> 958
   <212> DNA
   <213> Malus *sieboldii*
<400> 6
   atcacttgaa cacaccaatc cttgcacttg tagctacaat tctaagtttc cattttccaa      60
   catcccatca cattgttcaa aaatatcatc agcctcgagt tagggtttat tatccttcgt     120
   gaccctcttt ttagtatttg gttctttttg tatctcacca aaagaaaata tattaccagc     180
   tgtttacacc aaattaaata gtaaaaagaa agcatcaatg gctttaaaaa cacagttgtt     240
   gtggtcattt gttgtggttt ttgttgtttc cttcagtaca acttcatgtt ctggtagtag     300
   tttccaggag gtcaacgcgc ttcatagtta cgttgaccat gttgatgata aagagtccgg     360
   ctgtaattct aggacttatc cttcatacac ggacaccatt attgaaggtt taaagttcat     420
   ggaattgatc aggccaataa ctcagctctt cagttcaagg aagcacaaca caatcaccgg     480
   tgggatagca acatcatcag ctcgggccaa aaccattagc gtcgacgatt ttggagctaa     540
   aggggatggt gctgatgaca cacaggcatt tgagaaggca tggaaggcag cttgttcttc     600
   cagtggagct atggttcttg tggtaccgca gaagaactat cttgttgggc caattgaatt     660
   ctcaggccca tgcaaatctc gacttacact gcagatttat ggaaccatag aagcatcaga     720
   agaccgatca atctacaaag acacagacca ctggctcatc tttgacaatg tccaaaactt     780
   ggtagttgtt ggtcctggaa caatcaatgg caatggaaac atctggtgga aaaactcatg     840
   caaaataaaa cctcagcccc cttgcgatac atacgccccc atggctgtaa ccttcgacaa     900
   gtgcaataac ttggtggtga agaatctgaa tatccgagac gcacaacaaa tgcatgtc       958
<210> 7
   <211> 653
   <212> DNA
   <213> Malus *sieboldii*
<400> 7
   tcatgtgtca aatacccaga atatcactat ctccagctcc gttataggaa caggtgatga      60
   ctgtatttct attgtgagtg ggtcccaaag agttcaagcc acagacatta cttgtggacc     120
   aggccatgga atcagtattg gtagcttggg agaagacggc tcaaaagatc atgtttcagg     180
   agtatttgtg aatggagcta agctttcagg aacctccaat ggactccgga tcaagacgtg     240
   ggagggaggc tcaggcagtg caaccaacat tgttttccag aatgtgcaaa tgaacgatgt     300
   cgccaacccc atcatcatcg accagaacta ccgtgaccac aaaaccaaag attgcaaaca     360
   acagaaatcg gcggttcaag tgaaaaatgt gttgtacaaa aacataagag gaacgagtgc     420
   ttcccgcgac gcgataacgt tgaactgcag cccaagtgtt ccttgtcagg ggatcgtgct     480
   gcaaagtgtt caactgcaga atggaagagc tgaatgcaac aatgttaagc ctgcttacaa     540
   aggagttgtc tcccctagat gttaaaacct agggctcata attatgggca atgtgaaaca     600
   gcttatgcaa ttcttgtacg aattagcaca taaataattg tttgtttgta ata            653

## Description

### TECHNICAL FIELD

The present invention relates methods and compositions for producing plants with fruit with increased storage life.

### BACKGROUND ART

Post-harvest spoilage is a major problem for the fruit industry. It has been estimated that 10-20% of post-harvest fruit is lost through spoilage before reaching consumers. This spoilage results in increased cost of the non-spoiled fruit to the consumer. In addition fruit is often discarded by the consumer because of spoilage after purchase but before the fruit is eaten.

One of the main causes of spoilage is the natural ripening of fruit. As fruit ripens it tends to become softer and more susceptible to mechanical damage, as well as biological damage from necrophytic pathogens such as storage rots.

In addition to the problems associated with softening, the flesh of fruits such as apples often develop a "mealy" dry texture during post-harvest storage, which is unpopular with consumers. Mealy texture is believed to be a result of separation of cells in the flesh when the fruit is bitten, without associated rupture of cells and release of the apple's juice. This creates the impression of a dry, less juicy apple, as well as a less crunchy or crispy apple.

Water loss from fruit during storage is also a problem and can lead to fruit developing an unattractive shriveled appearance.

Various approaches have been used to attempt to address post-harvest deterioration of fruit. For example, genetic approaches have focused on manipulating the expression of ethylene biosynthesic genes such as ACC oxidase (e.g. in apple, Schaffer et al 2007; tomato, Hamilton et al 1990; and melon, Ayub et al 1996), and ACC synthase (Oeller et al 1991) and on genes that influence cell wall physiology such as pectin lyase (Santiago-Doménech et al 2008), expansin (Brummell et al 1999) and β-galactosidase (Carey et al 2001). However, to the applicants knowledge no fruits resulting from such approaches are currently commercially available.

Transgenic tomatoes in which expression of a polygalacturonase (PG) gene was reduced were commercialised as the well documented Flavr Savr tomatoes. However technical problems reportedly made it difficult to ship the delicate GE tomatoes without damage. Sale of Flavr Savr tomatoes was ultimately withdrawn.

An example of such a Transgenic tomato is given by Kramer et al "Postharvest evaluation of Transgenic tomatoes with reduced levels of polygalacturonase: processing, firmness and disease resistance", POSTHARVEST BIOLOGY AND TECHNOLOGY. (19920301), vol. 1, no. 3, PAGE 241 - 255.

From a scientific perspective tomato fruit with altered levels of polygalacturonase have been studied extensively. Tomato fruit containing an antisense PG gene (pTOM6) showed reduced depolymerization of pectin polymers in fruit (Smith et al., 1990). However "the firmness of the fruit throughout ripening was not altered in the transgenic samples when compared to controls" (Schuch et al 1991). Postharvest cracking, rates of infection, and the ability to withstand transport were improved in the antisense PG tomatoes. Many other studies on these transgenic lines support the role for PG in pectin depolymerization but not fruit softening (Carrington et al 1993, Cantu et al 2008; Langley et al 1994, Powell et al 1993)

Overexpression of PG in the ripening inhibited mutant *rin* background restored PG activity and pectin degradation in fruit. However, no significant effect on fruit softening, ethylene evolution, or color development was detected. The authors reported that "polygalacturonase was the primary determinant of cell wall polyuronide degradation, but suggested that this degradation was not sufficient for the induction of softening" (Giovannoni et al., 1989).

Further experiments where the pTOM6 gene was overexpressed in tobacco (*Nicotiana tabacum*; Osteryoung et al., 1990) showed that the tomato protein was properly processed and localized in the cell walls of leaves in tobacco. The enzyme showed activity when extracted from transgenic tobacco leaves and tested against tobacco cell wall extracts in vitro. However, no changes in leaf phenotype were observed, nor were there any alterations to the pectins in the tobacco cell walls *in vivo.*

Together these results suggested to researchers that PG only had role in pectin depolymerization primarily in fruit but the enzyme did not have an affect on fruit softening.

Thus in spite of such substantial research the problem of post-harvest softening has not been overcome for most fruits and particularly in apple. Apple (*Malus domestica* Borkh. cv Royal Gala) ripens very differently than tomato and many other fruits because cell wall swelling is not one of the cell wall modifications occurring during apple ripening (Redgwell et al., 1997). There is minimal change in viscosity of cell walls, and minimal pectin solubilization or degradation during fruit ripening. It would therefore be of benefit to provide improved or alternative methods to addressing post-harvest softening in apples and other fruit.

Wakasa et al ("Low expression of an endopolygalacturonase gene in apple fruit with long-term storage potential.", POSTHARVEST BIOLOGY AND TECHNOLOGY., (200602), vol. 39, no. 2, PAGE 193 - 198) investigated how softening patterns differ among cultivars using northern blot analysis and real-time quantative RT-PCR analysis. They concluded cultivars that remain firm revealed very weak or transient expression of *MdPG1*, irrespective of ethylene production rate and transcription levels of other genes.

It is an object of the invention to provide improved methods and compositions for producing plants with fruit having increased post-harvest storage life, or at least to provide the public with a useful choice.

### SUMMARY OF THE INVENTION

In a first aspect the invention provides a method for increasing post-harvest storage life and firmness of fruit of a plant from a Rosaceae species during, or after, post-harvest storage, relative to the fruit of a control plant under the same conditions, the method comprising reducing the expression or activity in the plant, of a polypeptide with the amino acid sequence of SEQ ID NO: 1, or a variant of the polypeptide with at least 70% identity to the amino acid sequence of SEQ ID NO: 1 over the entire length of SEQ ID NO: 1, wherein the method comprises the step of introducing a polynucleotide into a plant cell, or plant, to effect reducing the expression of the polypeptide or variant, wherein the polynucleotide comprises at least one of:
i) a sequence with at least 70% identity to part of an endogenous gene, or nucleic acid, that encodes the polypeptide or variant thereof, and
ii) a sequence that hybridizes under stringent conditions to part of an endogenous gene, or nucleic acid, encoding the polypeptide or variant thereof,
wherein the variant has polygalacturonase activity.

In one embodiment the fruit have at least one of:
a) reduced water loss,
b) reduced cell separation,
c) increased juiciness,
d) increased crispiness,
e) increased waxiness, and
f) reduced susceptibility to necrophytic pathogens, during, or after, post-harvest storage.

Preferably in addition to increased firmness, the fruit also show at least one of a) to f). Preferably, in addition to increased firmness, the fruit preferably have at least two, more prerably at least three, more prerably at least four, more prerably at least five, and most prerably all, of a) to f).

In a further embodiment the variant comprises the sequence of SEQ ID NO: 2.

In a further embodiment the variant comprises the sequence of SEQ ID NO: 3.

### Reducing expression of the polypeptide by introducing a polynucleotide

In a further embodiment the method comprises the step of introducing a polynucleotide into a plant cell, or plant, to effect reducing the expression of the polypeptide or variant.

### Targetting gene encoding polypeptide using complementary polynucleotide

In a further embodiment the polynucleotide comprises a sequence with at least 70% identity to part of an endogenous gene, or nucleic acid, that encodes the polypeptide or variant thereof.

In a further embodiment the polynucleotide comprises a sequence that hybridises under stringent conditions to part of an endogenous gene, or nucleic acid, encoding the polypeptide, or a variant of the polypeptide.

The part of the endogenous gene may include part of an element selected from the promoter, a 5' untranslated sequence (UTR), an exon, an intron, a 3' UTR or the terminator of the gene, or a may span more than one of such elements.

In a further embodiment the endogenous gene has at least 70% identity to the sequence of SEQ ID NO: 4.

In a further embodiment the endogenous gene has the sequence of SEQ ID NO: 4.

In a further embodiment the endogenous nucleic acid comprises a sequence with at least 70% identity to the sequence of SEQ ID NO: 5.

In a further embodiment the endogenous nucleic acid comprises the sequence of SEQ ID NO: 5.

In a further embodiment the endogenous nucleic acid comprises the sequence of SEQ ID NO: 6.

In a further embodiment the endogenous nucleic acid comprises the sequence of SEQ ID NO: 7.

In a further embodiment the polynucleotide comprises at least 15 contiguous nucleotides that are at least 70% identical to part of the endogenous gene or nucleic acid.

In a further embodiment the polynucleotide comprises at least 15 contiguous nucleotides that hybridise under stringent conditions to the endogenous gene or nucleic acid.

In a further embodiment the polynucleotide is introduced into the plant as part of a genetic construct.

In a further embodiment the genetic construct is an expression construct comprising a promoter operably linked to the polynucleotide.

In a further embodiment the polynucleotide in a sense orientation relative to the promoter.

In a further embodiment the polynucleotide in an antisense orientation relative to the promoter.

In a further embodiment the expression construct is an RNAi construct.

In a further embodiment the polynucleotide and a sequence complimentary to the polynucleotide are included in the RNAi construct to form the hairpin loop of the RNAi construct.

In a further embodiment the polynucleotide and a sequence complimentary to the polynucleotide are included in the RNAi construct to form a double stranded RNA when the polynucleotide and sequence complimentary thereto are transcribed.

In a further embodiment the polynucleotide comprises at least 15 contiguous nucleotides of a sequence with at least 70% identity to the sequence of SEQ ID NO: 4.

In a further embodiment the polynucleotide comprises at least 15 contiguous nucleotides of the sequence of SEQ ID NO: 4.

In a further embodiment the polynucleotide comprises at least 15 contiguous nucleotides of a sequence with at least 70% identity to the sequence of SEQ ID NO: 5.

In a further embodiment the polynucleotide comprises at least 15 contiguous nucleotides of the sequence of SEQ ID NO: 5.

In a further embodiment the polynucleotide comprises at least 15 contiguous nucleotides of the sequence of SEQ ID NO: 6.

In a further embodiment the polynucleotide comprises at least 15 contiguous nucleotides of the sequence of SEQ ID NO: 7.

In a further embodiment a plant with reduced expression of the polypeptide is regenerated from the plant cell.

Also described herein is a plant produced by the method.

### Silencing constructs and cells and plants containing the silencing constructs

Also described herein is an expression construct comprising a promoter operably linked to a polynucleotide comprising a fragment of at least 15 contiguous nucleotides of a sequence with at least 70% identity to any one of SEQ ID NO: 4, 5, 6 and 7, wherein the sequence with 70% identity to any one of SEQ ID NO: 4, 5, 6 and 7, encodes a polypeptide with polygalacturonase activity.

In one embodiment the polynucleotide comprises a fragment of at least 15 contiguous nucleotides any one of SEQ ID NO: 4, 5, 6 and 7.

In a further embodiment the polynucleotide in an antisense orientation relative to the promoter.

In a further embodiment the expression construct is an RNAi construct.

In a further embodiment the polynucleotide and a sequence complimentary to the polynucleotide are included in the RNAi construct to form the hairpin loop of the RNAi construct.

In a further embodiment the polynucleotide and a sequence complimentary to the polynucleotide are included in the RNAi construct to form a double stranded RNA when the polynucleotide and sequence complimentary thereto are transcribed.

Also described herein is a plant cell, or plant, comprising an expression construct of the invention.

Preferably the plant cell of plant has modified expression of the endogenous nucleic acid corresponding to the polynucleotide.

Preferably the endogenous nucleic acid encodes a polypeptide with polygalacturonase activity.

In further embodiment the plant has, or is capable of producing, fruit with increased post-harvest storage life.

In addition to increased firmness the fruit may have at least one of:
a) reduced water loss,
b) reduced cell separation,
c) increased juiciness,
d) increased crispiness,
e) increased waxiness, and
f) reduced susceptibility to necrophytic pathogens, during, or after, post-harvest storage.

In addition to increased firmness, the fruit may have at least two, more prerably at least three, more prerably at least four, more prerably at least five, and most prerably all, of b) to g).

Also described herein is a method for selecting a plant with, or capable of producing, fruit having increased post-harvest storage life, the method comprising testing of a plant for altered expression of a polynucleotide encoding a polypeptide with at least 70% identity to the sequence of SEQ ID NO: 1.

Also described herein is a method for selecting a plant with, or capable of producing, fruit having increased post-harvest storage life, the method comprising testing of a plant for altered expression of a polypeptide with at least 70% identity to the sequence of SEQ ID NO: 1.

In one embodiment of the above two instances, the polypeptide has the sequence of SEQ ID NO: 2.

In a further embodiment of the above two instances, the polypeptide has the sequence of SEQ ID NO: 3.

Also described herein is a group or population of plants selected by the method of the invention.

Also described herein is an isolated polynucleotide encoding a polypeptide comprising a sequence of SEQ ID NO: 2 or 3 or a variant thereof wherein the variant has polygalacturonase activity.

In one embodiment the variant comprises a sequence with at least 90% identity to SEQ ID NO: 2:

In a further embodiment the polypeptide comprises the sequence of SEQ ID NO: 2.

In one embodiment the variant comprises a sequence with at least 90% identity to SEQ ID NO: 3:

In a further embodiment the polypeptide comprises the sequence of SEQ ID NO: 3.

Also described herein is an isolated polynucleotide comprising the sequence of SEQ ID NO: 6 or 7, or a variant thereof wherein the variant encodes a polypeptide with polygalacturonase activity.

In one embodiment the variant comprises a sequence with at least 70% sequence identity to the sequence of SEQ ID NO: 6.

In one embodiment the polynucleotide comprises the sequence of any one of SEQ ID NO: 6.

In one embodiment the variant comprises a sequence with at least 70% sequence identity to the sequence of SEQ ID NO: 7.

In one embodiment the polynucleotide comprises the sequence of any one of SEQ ID NO: 7.

Also described herein is a genetic construct which comprises a polynucleotide used in the invention.

Also described herein is an expression construct which comprises a polynucleotide used in the invention.

Also described herein is an RNAi construct which comprises a polynucleotide used in the invention.

Also described herein is a vector comprising an expression construct, genetic construct or RNAi construct used in the invention.

Also described herein is a host cell genetically modified to express a polynucleotide used in the invention.

Also described herein is a host cell comprising an expression construct or genetic construct used in the invention.

Preferably the host cell is a plant cell. Preferably the plant cell is part of a plant.

Also described herein is a plant cell genetically modified to express a polynucleotide used in the invention.

Also described herein is a plant cell which comprises an expression construct used in the invention or the genetic construct used in the invention.

The polynucleotides and variants of polynucleotides used in the methods of the invention, may be derived from any species. The polynucleotides and variants may also be recombinantly produced and also may be the products of "gene shuffling' approaches.

In one embodiment the polynucleotide or variant, is derived from a plant species.

In a further embodiment the polynucleotide or variant, is derived from a gymnosperm plant species.

In a further embodiment the polynucleotide or variant, is derived from an angiosperm plant species.

In a further embodiment the polynucleotide or variant, is derived from a from dicotyledonous plant species.

The plant cells and plants described herein, including those from which the polynucleotides, variant polynucleotides, polypeptide and variant polypeptides are derived, may be from any fruit species.

In one embodiment the fruit are from Rosaceae species.

Preferred Rosaceae genera include *Exochorda, Maddenia, Oemleria, Osmaronia, Prinsepia, Prunus, Maloideae, Amelanchier, Aria, Aronia, Chaenomeles, Chamaemespilus, Cormus, Cotoneaster, CrataegusOsmaronia, Prinsepia, Prunus, Maloideae, Amelanchier, Aria, Aronia, Chaenomeles, Chamaemespilus, Cormus, Cotoneaster, Crataegu, Cydonia, Dichotomanthes, Docynia, Docyniopsis, Eriobotrya, Eriolobus, Heteromeles, Kageneckia, Lindleya, Malacomeles, Malus, Mespilus, Osteomeles, Peraphyllum, Photinia, Pseudocydonia, Pyracantha, Pyrus, Rhaphiolepis, Sorbus, Stranvaesia, Torminalis, Vauquelinia, Rosoideae, Acaena, Acomastylis, Agrimonia, Alchemilla, Aphanes, Aremonia, Bencomia, Chamaebatia, Cliffortia, Coluria, Cowania, Dalibarda, Dendriopoterium, Dryas, Duchesnea, Erythrocoma, Fallugia, Filipendula, Fragaria, Geum, Hagenia, Horkelia, Ivesia, Kerria, Leucosidea, Marcetella, Margyricarpus, Novosieversia,Oncostylus, Polylepis, Potentilla, Rosa, Rubus, Sanguisorba, Sarcopoterium, Sibbaldia, Sieversia, Taihangia, Tetraglochin, Waldsteinia, Rosaceae incertae sedis, Adenostoma, Aruncus, Cercocarpus, Chamaebatiarica, Chamaerhodos, Gillenia, Holodiscus, Lyonothamnus, Neillia, Neviusia, Physocarpus, Purshia, Rhodotypos, Sorbaria, Spiraea and Stephanandra.*

Preferred Rosaceae species include: *Exochorda giraldii, Exochorda racemosa, Exochorda,Exochorda giraldii, Exochorda racemosa, Exochorda serratifolia, Maddenia hypoleuca, Oemleria cerasiformis, Osmaronia cerasiformis, Prinsepia sinensis, Prinsepia uniflora, Prunus alleghaniensis, Prunus americana, Prunus andersonii, Prunus angustifolia, Prunus apetala, Prunus argentea, Prunus armeniaca, Primus avium, Prunus bifrons, Prunus brigantina, Prunus bucharica, Prunus buergeriana, Prunus campanulata, Prunus caroliniana, Prunus cerasifera, Prunus cerasus, Prunus choreiana, Prunus cocomilia, Prunus cyclamina, Prunus davidiana, Prunus debilis, Prunus domestica, Prunus dulcis, Prunus emarginata, Prunus fasciculata, Prunus ferganensis, Prumus fordiana, Prunus fremontii, Prunus fruticosa, Prunus geniculata, Prunus glandulosa, Prunus gracilis, Prunus grayana, Prunus hortulana, Prunus ilicifolia, Prunus incise, Prunus jacquemontii, Prunus japonica, Prunus kuramica, Prunus laurocerasus, Prunus leveilleana, Prunus lusitanica, Prunus maackii, Prunus mahaleb, Prunus mandshurica, Prunus maritima, Prunus maximowiczii, Prunus mexicana, Prunus microcarpa, Prunus mira, Prunus mume, Prunus munsoniana, Prunus nigra, Prunus nipponica, Prunus padus, Prunus pensylvanica, Prunus persia, Prunus petunnikowii, Prunus prostrata, Prunus pseudocerasus, Prunus pumila, Prunus rivularis, Prunus salicina, Prunus sargentii, Prunus sellowii, Prunus serotina, Prunus serrulata, Prunus sibirica, Prunus simonii, Prunus spinosa,Prunus spinulosa, Prunus subcordata, Prunus subhirtella, Prunus takesimensis, Prunus tenella,Prunus texana,Prunus tomentosa,Prunus tschonoskii,Prunus umbellata, Prunus verecunda,Prunus virginiana,Prunus webbii, Prunus x yedoensis, Prunus zippeliana, Prunus sp. BSP-2004-1, Prunus sp. BSP-2004-2,Prunus sp. EB-2002, Amelanchier alnifolia, Amelanchier arborea, Amelanchier asiatica, Amelanchier bartramiana, Amelanchier canadensis, Amelanchier cusickii, Amelanchier fernaldii, Amelanchier florida, Amelanchier humilis, Amelanchier intermedia, Amelanchier laevis, Amelanchier lucida, Amelanchier nantucketensis, Amelanchier pumila, Amelanchier quinti-martii, Amelanchier sanguinea, Amelanchier stolonifera, Amelanchier utahensis, Amelanchier wiegandii, Amelanchier x neglecta, Amelanchier bartramiana x Amelanchier sp. 'dentata', Amelanchier sp. 'dentata', Amelanchier sp. 'erecta', Amelanchier sp. 'erecta' x Amelanchier laevis, Amelanchier sp. 'serotina', Aria alnifolia, Aronia prunifolia, Chaenomeles cathayensis, Chaenomeles speciosa, Chamaemespilus alpina, Cormus domestica, Cotoneaster apiculatus, Cotoneaster lacteus, Cotoneaster pannosus, Crataegus azarolus, Crataegus columbiana, Crataegus crus-galli, Crataegus curvisepala, Crataegus laevigata, Crataegus mollis, Crataegus monogyna, Crataegus nigra, Crataegus rivularis, Crataegus sinaica, Cydonia oblonga, Dichotomanthes tristaniicarpa, Docynia delavayi, Docyniopsis tschonoskii, Eriobotrya japonica, Eriobotrya prinoides, Eriolobus trilobatus, Heteromeles arbutifolia, Kageneckia angustifolia, Kageneckia oblonga, Lindleya mespiloides, Malacomeles denticulata, Malus angustifolia, Malus asiatica, Malus baccata, Malus coronaria, Malus doumeri, Malus florentina, Malus floribunda, Malus fusca, Malus halliana, Malus honanensis, Malus hupehensis, Malus ioensis, Malus kansuensis, Malus mandshurica, Malus micromalus, Malus niedzwetzkyana, Malus ombrophilia, Malus orientalis, Malus prattii, Malus prunifolia, Malus pumila, Malus sargentii, Malus sieboldii, Malus sieversii, Malus sylvestris,Malus toringoides, Malus transitoria, Malus trilobata, Malus tschonoskii, Malus x domestica, Malus x domestica x Malus sieversii, Malus x domestica x Pyrus communis, Malus xiaojinensis, Malus yunnanensis, Malus sp., Mespilus germanica, Osteomeles anthyllidifolia, Osteomeles schwerinae, Peraphyllum ramosissimum, Photinia fraseri, Photinia pyrifolia, Photinia serrulata, Photinia villosa, Pseudocydonia sinensis, Pyracantha coccinea, Pyracantha fortuneana, Pyrus calleryana, Pyrus caucasica, Pyrus communis, Pyrus elaeagrifolia, Pyrus hybrid cultivar, Pyrus pyrifolia, Pyrus salicifolia, Pyrus ussuriensis, Pyrus x bretschneideri, Rhaphiolepis indica, Sorbus americana, Sorbus aria, Sorbus aucuparia, Sorbus california, Sorbus commixta, Sorbus hupehensis, Sorbus scopulina, Sorbus sibirica, Sorbus torminalis, Stranvaesia davidiana, Torminalis clusii, Vauquelinia californica, Vauquelinia corymbosa, Acaena anserinifolia, Acaena argentea, Acaena caesiiglauca, Acaena cylindristachya, Acaena digitata, Acaena echinata, Acaena elongata, Acaena eupatoria, Acaena fissistipula, Acaena inermis, Acaena laevigata, Acaena latebrosa, Acaena lucida, Acaena macrocephala, Acaena magellanica, Acaena masafuerana, Acaena montana, Acaena multifida, Acaena novaezelandiae, Acaena ovalifolia, Acaena pinnatifida, Acaena splendens, Acaena subincisa, Acaena x anserovina, Acomastylis elata, Acomastylis rossii, Acomastylis sikkimensis, Agrimonia eupatoria, Agrimonia nipponica, Agrimonia parviflora, Agrimonia pilosa, Alchemilla alpina, Alchemilla erythropoda, Alchemilla japonica, Alchemilla mollis, Alchemilla vulgaris, Aphanes arvensis, Aremonia agrimonioides, Bencomia brachystachya, Bencomia caudata, Bencomia exstipulata, Bencomia sphaerocarpa, Chamaebatia foliolosa, Cliffortia burmeana, Cliffortia caneata, Cliffortia dentata, Cliffortia graminea, Cliffortia heterophylla, Cliffortia nitidula, Cliffortia odorata, Cliffortia ruscifolia, Cliffortia sericea, Coluria elegans, Coluria geoides, Cowania stansburiana, Dalibarda repens, Dendriopoterium menendezii, Dendriopoterium pulidoi, Dryas drummondii, Dryas octopetala, Duchesnea chrysantha, Duchesnea indica, Erythrocoma triflora, Fallugia paradoxa, Filipendula multijuga Filipendula purpurea, Filipendula ulmaria, Filipendula vulgaris, Fragaria chiloensis*, *Fragaria daltoniana, Fragaria gracilis, Fragaria grandiflora, Fragaria iinumae, Fragaria moschata, Fragaria nilgerrensis, Fragaria nipponica, Fragaria nubicola, Fragaria orientalis, Fragaria pentaphylla, Fragaria vesca, Fragaria virginiana, Fragaria viridis*, *Fragaria x ananassa, Fragaria sp. CFRA 538, Fragaria sp.,Geum andicola, Geum borisi*, *Geum bulgaricum, Geum calthifolium, Geum chiloense, Geum geniculatum, Geum heterocarpum, Geum macrophyllum, Geum montanum, Geum reptans, Geum rivale, Geum schofieldii, Geum speciosum, Geum urbanum, Geum vernum, Geum sp. 'Chase 2507 K*',*Hagenia abyssinica,Horkelia cuneata, Horkelia fusca, Ivesia gordoni, Kerria japonica, Leucosidea sericea, Marcetella maderensis, Marcetella moquiniana*, *Margyricarpus pinnatus, Margyricarpus setosus, Novosieversia glacialis, Onccostylus cockaynei, Oncoslylus leiospermus, Polylepis australis, Polylepis besseri, Polylepis crista-galli, Polylepis hieronymi, Polylepis incana, Polylepis lanuginosa, Polylepis multijuga, Polylepis neglecta, Polylepis pauta, Polylepis pepei, Polylepis quadrijuga, Polylepis racemosa, Polylepis reticulata, Polylepis rugulosa, Polylepis sericea, Polylepis subsericans, Polylepis tarapacana, Polylepis tomentella, Polylepis weberbaueri, Potentilla anserina, Potentilla arguta, Potentilla bifurca, Potentilla chinensis, Potentilia dickinsii, Potentilla erecta, Potentilla fragarioides, Potentilla fruticosa, Potentilla indica, Potentilla micrantha, Potentilla multifida, Potentilla nivea, Potentilla norvegica, Potentilla palustris, Potentilla peduncularis, Potentilla reptans, Potentilla salesoviana, Potentilla stenophylla, Potentilla tridentata, Rosa abietina, Rosa abyssinica, Rosa acicularis, Rosa agrestis, Rosa alba, Rosa alba x Rosa corymbifera, Rosa altaica, Rosa arkansana, Rosa arvensis, Rosa banksiae, Rosa beggeriana, Rosa blanda, Rosa bracteata, Rosa brunonii, Rosa caesia, Rosa californica, Rosa canina*, *Rosa carolina, Rosa chinensis, Rosa cinnamomea, Rosa columnifera, Rosa corymbifera, Rosa cymosa, Rosa davurica, Rosa dumalis, Rosa ecae, Rosa eglanteria, Rosa elliptica, Rosa fedtschenkoana, Rosa foetida, Rosa foliolosa, Rosa gallica, Rosa gallica x Rosa dumetorum, Rosa gigantea, Rosa glauca, Rosa helenae, Rosa henryi, Rosa hugonis, Rosa hybrid cultivar, Rosa inodora, Rosa jundzillii, Rosa laevigata, Rosa laxa, Rosa luciae, Rosa majalis, Rosa marretii, Rosa maximowicziana, Rosa micrantha, Rosa mollis, Rosa montana, Rosa moschata, Rosa moyesii, Rosa multibracteata, Rosa multiflora, Rosa nitida, Rosa odorata, Rosa palustris, Rosa pendulina, Rosa persica, Rosa phoenicia, Rosa platyacantha, Rosa primula, Rosa pseudoscabriuscula, Rosa roxburghii, Rosa rubiginosa, Rosa rugosa, Rosa sambucina, Rosa sempervirens, Rosa sericea, Rosa sertata, Rosa setigera, Rosa sherardii*, *Rosa sicula, Rosa spinosissima, Rosa stellata, Rosa stylosa, Rosa subcanina, Rosa subcollina, Rosa suffulta, Rosa tomentella, Rosa tomentosa, Rosa tunquinensis, Rosa villosa, Rosa virginiana, Rosa wichurana, Rosa willmottiae, Rosa woodsii; Rosa x damascena, Rosa x fortuniana, Rosa x macrantha, Rosa xanthina, Rosa sp., Rubus alceifolius, Rubus allegheniensis, Rubus alpinus, Rubus amphidasys, Rubus arcticus, Rubus argutus, Rubus assamensis, Rubus australis*, *Rubus bifrons, Rubus caesius, Rubus caesius x Rubus idaeus, Rubus canadensis, Rubus canescens, Rubus caucasicus, Rubus chamaemorus, Rubus corchorifolius, Rubus crataegifolius, Rubus cuneifolius, Rubus deliciosus, Rubus divaricatus, Rubus ellipticus, Rubus flagellaris, Rubus fruticosus, Rubus geoides, Rubus glabratus, Rubus glaucus, Rubus gunnianus, Rubus hawaiensis, Rubus hawaiensis x Rubus rosifolius, Rubus hispidus*, *Rubus hochstetterorum, Rubus humulifolius, Rubus idaeus, Rubus lambertianus, Rubus lasiococcus, Rubus leucodermis, Rubus lineatus, Rubus macraei, Rubus maximiformis, Rubus minusculus, Rubus moorei, Rubus multibracteatus, Rubus neomexicanus, Rubus nepalensis, Rubus nessensis, Rubus nivalis*, *Rubus niveus, Rubus nubigenus, Rubus occidentalis, Rubus odoratus, Rubus palmatus, Rubus parviflorus, Rubus parvifolius, Rubus parvus, Rubus pectinellus, Rubus pedatus, Rubus pedemontanus, Rubus pensilvanicus, Rubus phoenicolasius, Rubus picticaulis, Rubus pubescens, Rubus rigidus, Rubus robustus, Rubus roseus*, *Rubus rosifolius, Rubus sanctus, Rubus sapidus, Rubus saxatilis, Rubus setosus, Rubus spectabilis, Rubus sulcatus, Rubus tephrodes, Rubus trianthus*, *Rubus tricolor, Rubus trifidus, Rubus trilobus, Rubus trivialis, Rubus ulmifolius, Rubus ursinus, Rubus urticifolius, Rubus vigorosus, Rubus sp. JPM-2004, Sanguisorba albiflora, Sanguisorba alpina, Sanguisorba ancistroides, Sanguisorba annua, Sanguisorba canadensis, Sanguisorba filiformis, Sanguisorba hakusanensis, Sanguisorba japonensis, Sanguisorba minor, Sanguisorba obtusa, Sanguisorba officinalis, Sanguisorba parviflora, Sanguisorba stipulata, Sanguisorba tenuifolia, Sarcopoterium spinosum, Sibbaldia procumbens, Sieversia pentapetala, Sieversia pusilla, Taihangia rupestris, Tetraglochin cristatum, Waldsteinia fragarioides, Waldsteinia geoides, Adenostoma fasciculatum, Adenostoma sparsifolium, Aruncus dioicus, Cercocarpus betuloides, Cercocarpus ledifolius, Chamaebatiaria millefolium, Chamaerhodos erecta, Gillenia stipulata, Gillenia trifoliata, Holodiscus discolor, Holodiscus microphyllus, Lyonothamnus floribundus, Neillia affinis, Neillia gracilis, Neillia sinensis, Neillia sparsiflora, Neillia thibetica, Neillia thyrsiflora, Neillia uekii, Neviusia alabamensis, Physocarpus alternans, Physocarpus amurensis, Physocarpus capitatus, Physocarpus malvaceus, Physocarpus monogynus, Physocarpus opulifolius, Purshia tridentata, Rhodotypos scandens, Sorbaria arborea, Sorbaria sorbifolia, Spiraea betulifolia, Spiraea cantoniensis, Spiraea densiflora, Spiraea japonica, Spiraea nipponica, Spiraea x vanhouttei, Spiraea sp., Stephanandra chinensis, Stephanandra incisa* and *Stephanandra tanakae.*

A particularly preferred genus is *Malus.*

Preferred *Malus* species include: *Malus aldenhamii Malus angustifolia, Malus asiatica, Malus baccata, Malus coronaria, Malus domestica, Malus doumeri, Malus florentina, Malus floribunda, Malus fusca, Malus halliana, Malus honanensis, Malus hupehensis, Malus ioensis, Malus kansuensis, Malus mandshurica, Malus micromalus, Malus niedzwetzkyana, Malus ombrophilia, Malus orientals, Malus prattii, Malus prunifolia, Malus pumila, Malus sargentii, Malus sieboldii, Malus sieversii, Malus sylvestris, Malus toringoides, Malus transitoria, Malus trilobata, Malus tschonoskii, Malus x domestic, Malus x domestica x Malus sieversii, Malus sylvestris, Malus x domestica x Pyrus communis, Malus xiaojinensis, Malus yunnanensis, Malus sp., Mespilus germanica,*

*A particularly preferred plant species is Malus domestica.*

Methods of the invention that include producing plants with increased firmness are particularly suitable for Malus species and fruit which don't have a melting texture when ripe such as Asian pear and non-melting peaches.

### DETAILED DESCRIPTION

The term "comprising" as used in this specification means "consisting at least in part of". When interpreting each statement in this specification that includes the term "comprising", features other than that or those prefaced by the term may also be present. Related terms such as "comprise" and "comprises" are to be interpreted in the same manner.

The invention provides methods and composition for producing plants with fruit having increased post-harvest storage life as defined in claim 1. The fruit have increased firmness, and may haveat least one of the following characteristics:
a) reduced water loss,
b) reduced cell separation,
c) increased juiciness,
d) increased crispiness,
e) increased waxiness, and
f) reduced susceptibility to necrophytic pathogens.

The increased firmness and terms, a) to f), are intended to be relative to terms. That is relative to the fruit of control plants under the same conditions.

For example a fruit with "increased firmness" is more firm than a control fruit (or fruit of a control plant) subjected to the same conditions during, or after, post-harvest storage. Thus "increased firmness" of the fruit of the method of the invention, is equivalent to "reduced softness" of the fruit of the method of the invention, when the control plant softens more during or after post-harvest storage than does the fruit of the method of the invention. It is not intended that "increased firmness" during, or after, post-harvest storage means that a fruit becomes more firm than it was before post-harvest storage.

Similarly each of the other terms a) to f) above are relative to control fruit under the same conditions.

The term "post-harvest storage" relates to storage of the fruit after harvesting from the plant/tree.

Typical post harvest storage conditions may include storage in controlled atmosphere, and/or modification of temperature (typically 0.5°C to 3°C) and/or application of growth regulators (such as 1-MCP).

Preferred post-harvest storage conditions depend on the region of growth and how long it is anticipated that the fruit need to be stored.

Exemplary post-harvest storage conditions for the purpose of the invention are 0.5°C for 10 weeks

### Polynucleotide and fragments

The term "polynucleotide(s)," as used herein, means a single or double-stranded deoxyribonucleotide or ribonucleotide polymer of any length but preferably at least 15 nucleotides, and include as non-limiting examples, coding and non-coding sequences of a gene, sense and antisense sequences complements, exons, introns, genomic DNA, cDNA, pre-mRNA, mRNA, rRNA, siRNA, miRNA, tRNA, ribozymes, recombinant polypeptides, isolated and purified naturally occurring DNA or RNA sequences, synthetic RNA and DNA sequences, nucleic acid probes, primers and fragments.

A "fragment" of a polynucleotide sequence provided herein is a subsequence of contiguous nucleotides that is capable of specific hybridization to a target of interest, e.g., a sequence that is at least 15 nucleotides in length. The fragments may comprise 15 nucleotides, preferably at least 16 nucleotides, more preferably at least 17 nucleotides, more preferably at least 18 nucleotides, more preferably at least 19 nucleotides, more preferably at least 20 nucleotides, more preferably at least 21 nucleotides, more preferably at least 22 nucleotides, more preferably at least 23 nucleotides, more preferably at least 24 nucleotides, more preferably at least 25 nucleotides, more preferably at least 26 nucleotides, more preferably at least 27 nucleotides, more preferably at least 28 nucleotides, more preferably at least 29 nucleotides, more preferably at least 30 nucleotides, more preferably at least 31 nucleotides, more preferably at least 32 nucleotides, more preferably at least 33 nucleotides, more preferably at least 34 nucleotides, more preferably at least 35 nucleotides, more preferably at least 36 nucleotides, more preferably at least 37 nucleotides, more preferably at least 38 nucleotides, more preferably at least 39 nucleotides, more preferably at least 40 nucleotides, more preferably at least 41 nucleotides, more preferably at least 42 nucleotides, more preferably at least 43 nucleotides, more preferably at least 44 nucleotides, more preferably at least 45 nucleotides, more preferably at least 46 nucleotides, more preferably at least 47 nucleotides, more preferably at least 48 nucleotides, more preferably at least 49 nucleotides, more preferably at least 50 nucleotides, more preferably at least 51 nucleotides, more preferably at least 52 nucleotides, more preferably at least 53 nucleotides, more preferably at least 54 nucleotides, more preferably at least 55 nucleotides, more preferably at least 56 nucleotides, more preferably at least 57 nucleotides, more preferably at least 58 nucleotides, more preferably at least 59 nucleotides, more preferably at least 60 nucleotides, more preferably at least 61 nucleotides, more preferably at least 62 nucleotides, more preferably at least 63 nucleotides, more preferably at least 64 nucleotides, more preferably at least 65 nucleotides, more preferably at least 66 nucleotides, more preferably at least 67 nucleotides, more preferably at least 68 nucleotides, more preferably at least 69 nucleotides, more preferably at least 70 nucleotides, more preferably at least 71 nucleotides, more preferably at least 72 nucleotides, more preferably at least 73 nucleotides, more preferably at least 74 nucleotides, more preferably at least 75 nucleotides, more preferably at least 76 nucleotides, more preferably at least 77 nucleotides, more preferably at least 78 nucleotides, more preferably at least 79 nucleotides, more preferably at least 80 nucleotides, more preferably at least 81 nucleotides, more preferably at least 82 nucleotides, more preferably at least 83 nucleotides, more preferably at least 84 nucleotides, more preferably at least 85 nucleotides, more preferably at least 86 nucleotides, more preferably at least 87 nucleotides, more preferably at least 88 nucleotides, more preferably at least 89 nucleotides, more preferably at least 90 nucleotides, more preferably at least 91 nucleotides, more preferably at least 92 nucleotides, more preferably at least 93 nucleotides, more preferably at least 94 nucleotides, more preferably at least 95 nucleotides, more preferably at least 96 nucleotides, more preferably at least 97 nucleotides, more preferably at least 98 nucleotides, more preferably at least 99 nucleotides, more preferably at least 100 nucleotides, more preferably at least 150 nucleotides, more preferably at least 200 nucleotides, more preferably at least 250 nucleotides, more preferably at least 300 nucleotides, more preferably at least 350 nucleotides, more preferably at least 400 nucleotides, more preferably at least 450 nucleotides and most preferably at least 500 nucleotides of contiguous nucleotides of a polynucleotide disclosed. A fragment of a polynucleotide sequence can be used in antisense, RNA interference (RNAi), gene silencing, triple helix or ribozyme technology, or as a primer, a probe, included in a microarray, or used in polynucleotide-based selection methods of the invention.

The term "primer" refers to a short polynucleotide, usually having a free 3'OH group, that is hybridized to a template and used for priming polymerization of a polynucleotide complementary to the target.

The term "probe" refers to a short polynucleotide that is used to detect a polynucleotide sequence, that is complementary to the probe, in a hybridization-based assay. The probe may consist of a "fragment" of a polynucleotide as defined herein.

### Polypeptides and fragments

The term "polypeptide", as used herein, encompasses amino acid chains of any length but preferably at least 5 amino acids, including full-length proteins, in which amino acid residues are linked by covalent peptide bonds. Polypeptides described herein may be purified natural products, or may be produced partially or wholly using recombinant or synthetic techniques.

The term may refer to a polypeptide, an aggregate of a polypeptide such as a dimer or other multimer, a fusion polypeptide, a polypeptide fragment, a polypeptide variant, or derivative thereof.

A "fragment" of a polypeptide is a subsequence of the polypeptide that performs a function that is required for the biological activity and/or provides three dimensional structure of the polypeptide. The term may refer to a polypeptide, an aggregate of a polypeptide such as a dimer or other multimer, a fusion polypeptide, a polypeptide fragment, a polypeptide variant, or derivative thereof capable of performing the above enzymatic activity.

The term "isolated" as applied to the polynucleotide or polypeptide sequences disclosed herein is used to refer to sequences that are removed from their natural cellular environment. An isolated molecule may be obtained by any method or combination of methods including biochemical, recombinant, and synthetic techniques.

The term "recombinant" refers to a polynucleotide sequence that is removed from sequences that surround it in its natural context and/or is recombined with sequences that are not present in its natural context.

A "recombinant" polypeptide sequence is produced by translation from a "recombinant" polynucleotide sequence.

The term "derived from" with respect to polynucleotides or polypeptides of the invention being derived from a particular genera or species, means that the polynucleotide or polypeptide has the same sequence as a polynucleotide or polypeptide found naturally in that genera or species. The polynucleotide or polypeptide, derived from a particular genera or species, may therefore be produced synthetically or recombinantly.

### Variants

As used herein, the term "variant" refers to polynucleotide or polypeptide sequences different from the specifically identified sequences, wherein one or more nucleotides or amino acid residues is deleted, substituted, or added. Variants may be naturally occurring allelic variants, or non-naturally occurring variants. Variants may be from the same or from other species and may encompass homologues, paralogues and orthologues. In certain embodiments, variants of the inventive polypeptides and polypeptides possess biological activities that are the same or similar to those of the inventive polypeptides or polypeptides. The term "variant" with reference to polypeptides and polypeptides encompasses all forms of polypeptides and polypeptides as defined herein.

### Polynucleotide variants

Variant polynucleotide sequences preferably exhibit at least 50%, more preferably at least 51%, more preferably at least 52%, more preferably at least 53%, more preferably at least 54%, more preferably at least 55%, more preferably at least 56%, more preferably at least 57%, more preferably at least 58%, more preferably at least 59%, more preferably at least 60%, more preferably at least 61%, more preferably at least 62%, more preferably at least 63%, more preferably at least 64%, more preferably at least 65%, more preferably at least 66%, more preferably at least 67%, more preferably at least 68%, more preferably at least 69%, more preferably at least 70%, more preferably at least 71%, more preferably at least 72%, more preferably at least 73%, more preferably at least 74%, more preferably at least 75%, more preferably at least 76%, more preferably at least 77%, more preferably at least 78%, more preferably at least 79%, more preferably at least 80%, more preferably at least 81 %, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, and most preferably at least 99% identity to a sequence described herein Identity is found over a comparison window of at least 20 nucleotide positions, preferably at least 50 nucleotide positions, more preferably at least 100 nucleotide positions, and most preferably over the entire length of polynucleotide described herein.

Polynucleotide sequence identity can be determined in the following manner. The subject polynucleotide sequence is compared to a candidate polynucleotide sequence using BLASTN (from the BLAST suite of programs, version 2.2.5 [Nov 2002]) in bl2seq (Tatiana A. Tatusova, Thomas L. Madden (1999), "Blast 2 sequences - a new tool for comparing protein and nucleotide sequences", FEMS Microbiol Lett. 174:247-250), which is publicly available from NCBI (ftp://ftp.ncbi.nih.gov/blast/). The default parameters of bl2seq are utilized except that filtering of low complexity parts should be turned off.

The identity of polynucleotide sequences may be examined using the following unix command line parameters:
bl2seq -i nucleotideseql -j nucleotideseq2 -F F -p blastn

The parameter -F F turns off filtering of low complexity sections. The parameter -p selects the appropriate algorithm for the pair of sequences. The bl2seq program reports sequence identity as both the number and percentage of identical nucleotides in a line "Identities = ".

Polynucleotide sequence identity may also be calculated over the entire length of the overlap between a candidate and subject polynucleotide sequences using global sequence alignment programs (e.g. Needleman, S. B. and Wunsch, C. D. (1970) J. Mol. Biol. 48, 443-453). A full implementation of the Needleman-Wunsch global alignment algorithm is found in the needle program in the EMBOSS package (Rice,P. Longden,I. and Bleasby,A. EMBOSS: The European Molecular Biology Open Software Suite, Trends in Genetics June 2000, vol 16, No 6. pp.276-277) which can be obtained from http://www.hgmp.mrc.ac.uk/Software/EMBOSS/. The European Bioinformatics Institute server also provides the facility to perform EMBOSS-needle global alignments between two sequences on line at http:/www.ebi.ac.uk/emboss/align/.

Alternatively the GAP program may be used which computes an optimal global alignment of two sequences without penalizing terminal gaps. GAP is described in the following paper: Huang, X. (1994) On Global Sequence Alignment. Computer Applications in the Biosciences 10, 227-235.

A preferred method for calculating polynucleotide % sequence identity is based on aligning sequences to be compared using Clustal X (Jeanmougin et al., 1998, Trends Biochem. Sci. 23, 403-5.)

Polynucleotide variants described herein also encompass those which exhibit a similarity to one or more of the specifically identified sequences that is likely to preserve the functional equivalence of those sequences and which could not reasonably be expected to have occurred by random chance. Such sequence similarity with respect to polypeptides may be determined using the publicly available bl2seq program from the BLAST suite of programs (version 2.2.5 [Nov 2002]) from NCBI (ftp://ftp.ncbi.nih.gov/blast/).

The similarity of polynucleotide sequences may be examined using the following unix command line parameters:
bl2seq -i nucleotideseq1 -j nucleotideseq2 -F F -p tblastx

The parameter -F F turns off filtering of low complexity sections. The parameter -p selects the appropriate algorithm for the pair of sequences. This program finds regions of similarity between the sequences and for each such region reports an "E value" which is the expected number of times one could expect to see such a match by chance in a database of a fixed reference size containing random sequences. The size of this database is set by default in the bl2seq program. For small E values, much less than one, the E value is approximately the probability of such a random match.

Variant polynucleotide sequences preferably exhibit an E value of less than 1 x 10⁻⁶ more preferably less than 1 x 10⁻⁹, more preferably less than 1 x 10⁻¹², more preferably less than 1 x 10⁻¹⁵, more preferably less than 1 x 10⁻¹⁸, more preferably less than 1 x 10⁻²¹, more preferably less than 1 x 10⁻³⁰, more preferably less than 1 x 10⁻⁴⁰, more preferably less than 1 x 10⁻⁵⁰, more preferably less than 1 x 10⁻⁶⁰, more preferably less than 1 x 10⁻⁷⁰, more preferably less than 1 x 10⁻⁸⁰, more preferably less than 1 x 10⁻⁹⁰ and most preferably less than 1 x 10⁻¹⁰⁰ when compared with any one of the specifically identified sequences.

Alternatively, variant polynucleotides used in the methods of the invention, hybridize to the specified polynucleotide sequences, or complements thereof under stringent conditions.

The term "hybridize under stringent conditions", and grammatical equivalents thereof, refers to the ability of a polynucleotide molecule to hybridize to a target polynucleotide molecule (such as a target polynucleotide molecule immobilized on a DNA or RNA blot, such as a Southern blot or Northern blot) under defined conditions of temperature and salt concentration. The ability to hybridize under stringent hybridization conditions can be determined by initially hybridizing under less stringent conditions then increasing the stringency to the desired stringency.

With respect to polynucleotide molecules greater than about 100 bases in length, typical stringent hybridization conditions are no more than 25 to 30° C (for example, 10° C) below the melting temperature (Tm) of the native duplex (see generally, Sambrook et al., Eds, 1987, Molecular Cloning, A Laboratory Manual, 2nd Ed. Cold Spring Harbor Press; Ausubel et al., 1987, Current Protocols in Molecular Biology, Greene Publishing,). Tm for polynucleotide molecules greater than about 100 bases can be calculated by the formula Tm = 81. 5 + 0. 41% (G + C-log (Na+). (Sambrook et al., Eds, 1987, Molecular Cloning, A Laboratory Manual, 2nd Ed. Cold Spring Harbor Press; Bolton and McCarthy, 1962, PNAS 84:1390). Typical stringent conditions for polynucleotide of greater than 100 bases in length would be hybridization conditions such as prewashing in a solution of 6X SSC, 0.2% SDS; hybridizing at 65°C, 6X SSC, 0.2% SDS overnight; followed by two washes of 30 minutes each in 1X SSC, 0.1% SDS at 65° C and two washes of 30 minutes each in 0.2X SSC, 0.1% SDS at 65°C.

With respect to polynucleotide molecules having a length less than 100 bases, exemplary stringent hybridization conditions are 5 to 10° C below Tm. On average, the Tm of a polynucleotide molecule of length less than 100 bp is reduced by approximately (500/oligonucleotide length)° C.

With respect to the DNA mimics known as peptide nucleic acids (PNAs) (Nielsen et al., Science. 1991 Dec 6;254(5037):1497-500) Tm values are higher than those for DNA-DNA or DNA-RNA hybrids, and can be calculated using the formula described in Giesen et al., Nucleic Acids Res. 1998 Nov 1;26(21):5004-6. Exemplary stringent hybridization conditions for a DNA-PNA hybrid having a length less than 100 bases are 5 to 10° C below the Tm.

Variant polynucleotides used in the methods of the invention, also encompasses polynucleotides that differ from the sequences of the invention but that, as a consequence of the degeneracy of the genetic code, encode a polypeptide having similar activity to a polypeptide encoded by a polynucleotide of the present invention. A sequence alteration that does not change the amino acid sequence of the polypeptide is a "silent variation". Except for ATG (methionine) and TGG (tryptophan), other codons for the same amino acid may be changed by art recognized techniques, e.g., to optimize codon expression in a particular host organism.

Polynucleotide sequence alterations resulting in conservative substitutions of one or several amino acids in the encoded polypeptide sequence without significantly altering its biological activity are also included in the invention. A skilled artisan will be aware of methods for making phenotypically silent amino acid substitutions (see, e.g., Bowie et al., 1990, Science 247, 1306).

Variant polynucleotides due to silent variations and conservative substitutions in the encoded polypeptide sequence may be determined using the publicly available bl2seq program from the BLAST suite of programs (version 2.2.5 [Nov 2002]) from NCBI (ftp://ftp.ncbi.nih.gov/blast/) via the tblastx algorithm as previously described.

The function of a variant polynucleotide or polypeptide described herein as a polygalacturonase may be assessed for example by expressing such a sequence in yeast and testing activity of the encoded protein as previously described for cell wall related proteins (van Rensberg et al 1994; Saladie et al 2006). Function of a variant may also be tested for its ability to alter polygalacturonase activity in plants, as described in (Hellens et al 2005). The function of variants in altering post-harvest storage life may be tested by methods described in this specification (e.g., in the Examples section) and by other methods known to those skilled in the art.

### Polypeptide variants

The term "variant" with reference to polypeptides encompasses naturally occurring, recombinantly and synthetically produced polypeptides. Variant polypeptide sequences preferably exhibit at least 50%, more preferably at least 51%, more preferably at least 52%, more preferably at least 53%, more preferably at least 54%, more preferably at least 55%, more preferably at least 56%, more preferably at least 57%, more preferably at least 58%, more preferably at least 59%, more preferably at least 60%, more preferably at least 61 %, more preferably at least 62%, more preferably at least 63%, more preferably at least 64%, more preferably at least 65%, more preferably at least 66%, more preferably at least 67%, more preferably at least 68%, more preferably at least 69%, more preferably at least 70%, more preferably at least 71%, more preferably at least 72%, more preferably at least 73%, more preferably at least 74%, more preferably at least 75%, more preferably at least 76%, more preferably at least 77%, more preferably at least 78%, more preferably at least 79%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, and most preferably at least 99% identity to a sequences described herein. Identity is found over a comparison window of at least 20 amino acid positions, preferably at least 50 amino acid positions, more preferably at least 100 amino acid positions, and most preferably over the entire length of a polypeptide described herein.

Polypeptide sequence identity can be determined in the following manner. The subject polypeptide sequence is compared to a candidate polypeptide sequence using BLASTP (from the BLAST suite of programs, version 2.2.5 [Nov 2002]) in bl2seq, which is publicly available from NCBI (ftp://ftp.ncbi.nih.gov/blast/). The default parameters of bl2seq are utilized except that filtering of low complexity regions should be turned off.

Polypeptide sequence identity may also be calculated over the entire length of the overlap between a candidate and subject polynucleotide sequences using global sequence alignment programs. EMBOSS-needle (available at http:/www.ebi.ac.uk/emboss/align/) and GAP (Huang, X. (1994) On Global Sequence Alignment. Computer Applications in the Biosciences 10,227-235.) as discussed above are also suitable global sequence alignment programs for calculating polypeptide sequence identity.

A preferred method for calculating polypeptide % sequence identity is based on aligning sequences to be compared using Clustal X (Jeanmougin et al., 1998, Trends Biochem. Sci. 23, 403-5.)

Polypeptide variants described herein also encompass those which exhibit a similarity to one or more of the specifically identified sequences that is likely to preserve the functional equivalence of those sequences and which could not reasonably be expected to have occurred by random chance. Such sequence similarity with respect to polypeptides may be determined using the publicly available bl2seq program from the BLAST suite of programs (version 2.2.5 [Nov 2002]) from NCBI (ftp://ftp.ncbi.nih.gov/blast/). The similarity of polypeptide sequences may be examined using the following unix command line parameters:
bl2seq -i peptideseq1 -j peptideseq2 -F F -p blastp

Variant polypeptide sequences preferably exhibit an E value of less than 1x10⁻⁶ more preferably less than 1x10⁻⁹, more preferably less than 1x10⁻¹², more preferably less than 1x10⁻¹⁵, more preferably less than 1x10⁻¹⁸, more preferably less than 1x10⁻²¹, more preferably less than 1x10⁻³⁰, more preferably less than 1 x 10⁻⁴⁰, more preferably less than 1x10⁻⁵⁰, more preferably less than 1x10⁻⁶⁰, more preferably less than 1x10⁻⁷⁰, more preferably less than 1x10⁻⁸⁰, more preferably less than 1x10⁻⁹⁰ and most preferably 1x10⁻¹⁰⁰ when compared with any one of the specifically identified sequences.

The parameter -F F turns off filtering of low complexity sections. The parameter -p selects the appropriate algorithm for the pair of sequences. This program finds regions of similarity between the sequences and for each such region reports an "E value" which is the expected number of times one could expect to see such a match by chance in a database of a fixed reference size containing random sequences. For small E values, much less than one, this is approximately the probability of such a random match.

Conservative substitutions of one or several amino acids of a described polypeptide sequence without significantly altering its biological activity are also envisaged. A skilled artisan will be aware of methods for making phenotypically silent amino acid substitutions (see, e.g., Bowie et al., 1990, Science 247, 1306).

### Constructs, vectors and components thereof

The term "genetic construct" refers to a polynucleotide molecule, usually double-stranded DNA, which may have inserted into it another polynucleotide molecule (the insert polynucleotide molecule) such as, but not limited to, a cDNA molecule. A genetic construct may contain the necessary elements that permit transcribing the insert polynucleotide molecule, and, optionally, translating the transcript into a polypeptide. The insert polynucleotide molecule may be derived from the host cell, or may be derived from a different cell or organism and/or may be a recombinant polynucleotide. Once inside the host cell the genetic construct may become integrated in the host chromosomal DNA. The genetic construct may be linked to a vector.

The term "vector" refers to a polynucleotide molecule, usually double stranded DNA, which is used to transport the genetic construct into a host cell. The vector may be capable of replication in at least one additional host system, such as *E. coli.*

The term "expression construct" refers to a genetic construct that includes the necessary elements that permit transcribing the insert polynucleotide molecule, and, optionally, translating the transcript into a polypeptide. An expression construct typically comprises in a 5' to 3' direction:
a) a promoter functional in the host cell into which the construct will be transformed,
b) the polynucleotide to be expressed, and
c) a terminator functional in the host cell into which the construct will be transformed.

The term "coding region" or "open reading frame" (ORF) refers to the sense strand of a genomic DNA sequence or a cDNA sequence that is capable of producing a transcription product and/or a polypeptide under the control of appropriate regulatory sequences. The coding sequence is identified by the presence of a 5' translation start codon and a 3' translation stop codon. When inserted into a genetic construct, a "coding sequence" is capable of being expressed when it is operably linked to promoter and terminator sequences.

"Operably-linked" means that the sequenced to be expressed is placed under the control of regulatory elements that include promoters, tissue-specific regulatory elements, temporal regulatory elements, enhancers, repressors and terminators.

The term "noncoding region" refers to untranslated sequences that are upstream of the translational start site and downstream of the translational stop site. These sequences are also referred to respectively as the 5' UTR and the 3' UTR. These regions include elements required for transcription initiation and termination and for regulation of translation efficiency.

Terminators are sequences, which terminate transcription, and are found in the 3' untranslated ends of genes downstream of the translated sequence. Terminators are important determinants of mRNA stability and in some cases have been found to have spatial regulatory functions.

The term "promoter" refers to nontranscribed cis-regulatory elements upstream of the coding region that regulate gene transcription. Promoters comprise cis-initiator elements which specify the transcription initiation site and conserved boxes such as the TATA box, and motifs that are bound by transcription factors.

A promoter may be homologous with respect to the polynucleotide to be expressed. This means that the promoter and polynucleotide are found operably linked in nature.

Alternatively the promoter may be heterologous with respect to the polynucleotide to be expressed. This means that the promoter and the polynucleotide are not found operably linked in nature.

A "transgene" is a polynucleotide that is taken from one organism and introduced into a different organism by transformation. The transgene may be derived from the same species or from a different species as the species of the organism into which the transgene is introduced.

An "inverted repeat" is a sequence that is repeated, where the second half of the repeat is in the complementary strand, e.g.,
(5')GATCTA.......TAGATC(3')
(3')CTAGAT.......ATCTAG(5')

Read-through transcription will produce a transcript that undergoes complementary base-pairing to form a hairpin structure provided that there is a 3-5 bp spacer between the repeated regions.

### Host cells

Host cells may be derived from, for example, bacterial, fungal, insect, mammalian or plant organisms.

A "transgenic plant" refers to a plant which contains new genetic material as a result of genetic manipulation or transformation. The new genetic material may be derived from a plant of the same species as the resulting transgenic plant or from a different species.

The applicants have surprisingly shown that plants transformed to reduce expression of the polypeptide of SEQ ID NO: 1, produce fruit with increased post-harvest storage life.

The plants have, or are capable of producing, fruit with the following characteristics:
a) increased firmness,
b) reduced water loss,
c) reduced cell separation,
d) increased juiciness,
e) increased crispiness,
f) increased waxiness, and
g) reduced susceptibility to necrophytic pathogens.

Described herein are expression constructs suitable for reducing the expression of the polypeptide of SEQ ID NO: 1 or variants thereof. Also described are plant cells and plants comprising the expression constructs.

Also provided are methods for producing, and selecting plants with with increased post-harvest storage life, relative to suitable control plants.

Suitable control plants include non-transformed plants of the same species or variety or plants transformed with control constructs.

### Methods for isolating or producing polynucleotides

The polynucleotide molecules of the invention can be isolated by using a variety of techniques known to those of ordinary skill in the art. By way of example, such polypeptides can be isolated through use of the polymerase chain reaction (PCR) described in Mullis et al., Eds. 1994 The Polymerase Chain Reaction, Birkhauser, incorporated herein by reference. The polypeptides of the invention can be amplified using primers, as defined herein, derived from the polynucleotide sequences of the invention.

Further methods for isolating the polynucleotides described herein invention include use of all, or portions of, the polypeptides having the sequence set forth herein as hybridization probes. The technique of hybridizing labelled polynucleotide probes to polynucleotides immobilized on solid supports such as nitrocellulose filters or nylon membranes, can be used to screen the genomic or cDNA libraries. Exemplary hybridization and wash conditions are: hybridization for 20 hours at 65°C in 5.0 X SSC, 0. 5% sodium dodecyl sulfate, 1 X Denhardt's solution; washing (three washes of twenty minutes each at 55°C) in 1. 0 X SSC, 1% (w/v) sodium dodecyl sulfate, and optionally one wash (for twenty minutes) in 0. 5 X SSC, 1% (w/v) sodium dodecyl sulfate, at 60°C. An optional further wash (for twenty minutes) can be conducted under conditions of 0.1X SSC, 1% (w/v) sodium dodecyl sulfate, at 60°C.

The polynucleotide fragments described herein may be produced by techniques well-known in the art such as restriction endonuclease digestion, oligonucleotide synthesis and PCR amplification.

A partial polynucleotide sequence may be used, in methods well-known in the art to identify the corresponding full length polynucleotide sequence. Such methods include PCR-based methods, 5'RACE (Frohman MA, 1993, Methods Enzymol. 218: 340-56) and hybridization- based method, computer/database -based methods. Further, by way of example, inverse PCR permits acquisition of unknown sequences, flanking the polynucleotide sequences disclosed herein, starting with primers based on a known region (Triglia et al., 1998, Nucleic Acids Res 16, 8186, incorporated herein by reference). The method uses several restriction enzymes to generate a suitable fragment in the known region of a gene. The fragment is then circularized by intramolecular ligation and used as a PCR template. Divergent primers are designed from the known region. In order to physically assemble full-length clones, standard molecular biology approaches can be utilized (Sambrook et al., Molecular Cloning: A Laboratory Manual, 2nd Ed. Cold Spring Harbor Press, 1987).

It may be beneficial, when producing a transgenic plant from a particular species, to transform such a plant with a sequence or sequences derived from that species. The benefit may be to alleviate public concerns regarding cross-species transformation in generating transgenic organisms. Additionally when down-regulation of a gene is the desired result, it may be necessary to utilise a sequence identical (or at least highly similar) to that in the plant, for which reduced expression is desired. For these reasons among others, it is desirable to be able to identify and isolate orthologues of a particular gene in several different plant species.

Variants (including orthologues) may be identified by the methods described.

### Methods for identifying variants

### Physical methods

Variant polypeptides may be identified using PCR-based methods (Mullis et al., Eds. 1994 The Polymerase Chain Reaction, Birkhauser). Typically, the polynucleotide sequence of a primer, useful to amplify variants of polynucleotide molecules of the invention by PCR, may be based on a sequence encoding a conserved region of the corresponding amino acid sequence.

Alternatively library screening methods, well known to those skilled in the art, may be employed (Sambrook et al., Molecular Cloning: A Laboratory Manual, 2nd Ed. Cold Spring Harbor Press, 1987). When identifying variants of the probe sequence, hybridization and/or wash stringency will typically be reduced relatively to when exact sequence matches are sought.

Polypeptide variants may also be identified by physical methods, for example by screening expression libraries using antibodies raised against polypeptides of the invention (Sambrook et al., Molecular Cloning: A Laboratory Manual, 2nd Ed. Cold Spring Harbor Press, 1987) or by identifying polypeptides from natural sources with the aid of such antibodies.

### Computer based methods

The variant sequences of the invention, including both polynucleotide and polypeptide variants, may also be identified by computer-based methods well-known to those skilled in the art, using public domain sequence alignment algorithms and sequence similarity search tools to search sequence databases (public domain databases include Genbank, EMBL, Swiss-Prot, PIR and others). See, e.g., Nucleic Acids Res. 29: 1-10 and 11-16, 2001 for examples of online resources. Similarity searches retrieve and align target sequences for comparison with a sequence to be analyzed (i.e., a query sequence). Sequence comparison algorithms use scoring matrices to assign an overall score to each of the alignments.

An exemplary family of programs useful for identifying variants in sequence databases is the BLAST suite of programs (version 2.2.5 [Nov 2002]) including BLASTN, BLASTP, BLASTX, tBLASTN and tBLASTX, which are publicly available from (ftp://ftp.ncbi.nih.gov/blast/) or from the National Center for Biotechnology Information (NCBI), National Library of Medicine, Building 38A, Room 8N805, Bethesda, MD 20894 USA. The NCBI server also provides the facility to use the programs to screen a number of publicly available sequence databases. BLASTN compares a nucleotide query sequence against a nucleotide sequence database. BLASTP compares an amino acid query sequence against a protein sequence database. BLASTX compares a nucleotide query sequence translated in all reading frames against a protein sequence database. tBLASTN compares a protein query sequence against a nucleotide sequence database dynamically translated in all reading frames. tBLASTX compares the six-frame translations of a nucleotide query sequence against the six-frame translations of a nucleotide sequence database. The BLAST programs may be used with default parameters or the parameters may be altered as required to refine the screen.

The use of the BLAST family of algorithms, including BLASTN, BLASTP, and BLASTX, is described in the publication of Altschul et al., Nucleic Acids Res. 25: 3389-3402, 1997.

The "hits" to one or more database sequences by a queried sequence produced by BLASTN, BLASTP, BLASTX, tBLASTN, tBLASTX, or a similar algorithm, align and identify similar portions of sequences. The hits are arranged in order of the degree of similarity and the length of sequence overlap. Hits to a database sequence generally represent an overlap over only a fraction of the sequence length of the queried sequence.

The BLASTN, BLASTP, BLASTX, tBLASTN and TBLASTX algorithms also produce "Expect" values for alignments. The Expect value (E) indicates the number of hits one can "expect" to see by chance when searching a database of the same size containing random contiguous sequences. The Expect value is used as a significance threshold for determining whether the hit to a database indicates true similarity. For example, an E value of 0.1 assigned to a polynucleotide hit is interpreted as meaning that in a database of the size of the database screened, one might expect to see 0.1 matches over the aligned portion of the sequence with a similar score simply by chance. For sequences having an E value of 0.01 or less over aligned and matched portions, the probability of finding a match by chance in that database is 1% or less using the BLASTN, BLASTP, BLASTX, tBLASTN or tBLASTX algorithm.

Multiple sequence alignments of a group of related sequences can be carried out with CLUSTALW (Thompson, J.D., Higgins, D.G. and Gibson, T.J. (1994) CLUSTALW: improving the sensitivity of progressive multiple sequence alignment through sequence weighting, positions-specific gap penalties and weight matrix choice. Nucleic Acids Research, 22:4673-4680, http://www-igbmc.ustrasbg.fr/BioInfo/ClustalW/Top.html) or T-COFFEE (Cedric Notredame, Desmond G. Higgins, Jaap Heringa, T-Coffee: A novel method for fast and accurate multiple sequence alignment, J. Mol. Biol. (2000) 302: 205-217)) or PILEUP, which uses progressive, pairwise alignments. (Feng and Doolittle, 1987, J. Mol. Evol. 25, 351).

Pattern recognition software applications are available for finding motifs or signature sequences. For example, MEME (Multiple Em for Motif Elicitation) finds motifs and signature sequences in a set of sequences, and MAST (Motif Alignment and Search Tool) uses these motifs to identify similar or the same motifs in query sequences. The MAST results are provided as a series of alignments with appropriate statistical data and a visual overview of the motifs found. MEME and MAST were developed at the University of California, San Diego.

PROSITE (Bairoch and Bucher, 1994, Nucleic Acids Res. 22, 3583; Hofmann et al., 1999, Nucleic Acids Res. 27, 215) is a method of identifying the functions of uncharacterized proteins translated from genomic or cDNA sequences. The PROSITE database (www.expasy.org/prosite) contains biologically significant patterns and profiles and is designed so that it can be used with appropriate computational tools to assign a new sequence to a known family of proteins or to determine which known domain(s) are present in the sequence (Falquet et al., 2002, Nucleic Acids Res. 30,235). Prosearch is a tool that can search SWISS-PROT and EMBL databases with a given sequence pattern or signature.

### Methods for isolating polypeptides

The polypeptides described herein, including variant polypeptides, may be prepared using peptide synthesis methods well known in the art such as direct peptide synthesis using solid phase techniques (e.g. Stewart et al., 1969, in Solid-Phase Peptide Synthesis, WH Freeman Co, San Francisco California, or automated synthesis, for example using an Applied Biosystems 431A Peptide Synthesizer (Foster City, California). Mutated forms of the polypeptides may also be produced during such syntheses.

The polypeptides and variant polypeptides may also be purified from natural sources using a variety of techniques that are well known in the art (e.g. Deutscher, 1990, Ed, Methods in Enzymology, Vol. 182, Guide to Protein Purification,).

Alternatively the polypeptides and variant polypeptides may be expressed recombinantly in suitable host cells and separated from the cells as discussed below.

### Methods for producing constructs and vectors

The genetic constructs described herein comprise one or more polynucleotide sequences described herein, and may be useful for transforming, for example, bacterial, fungal, insect, mammalian or plant organisms. The genetic constructs of the invention are intended to include expression constructs as herein defined.

Methods for producing and using genetic constructs and vectors are well known in the art and are described generally in Sambrook et al., Molecular Cloning: A Laboratory Manual, 2nd Ed. Cold Spring Harbor Press, 1987 ; Ausubel et al., Current Protocols in Molecular Biology, Greene Publishing, 1987).

### Methods for producing host cells comprising polynucleotides, constructs or vectors

Described herein is a host cell which comprises a genetic construct or vector described herein.

Host cells comprising genetic constructs, such as expression constructs, of the invention are useful in methods well known in the art (e.g. Sambrook et al., Molecular Cloning : A Laboratory Manual, 2nd Ed. Cold Spring Harbor Press, 1987 ; Ausubel et al., Current Protocols in Molecular Biology, Greene Publishing, 1987) for recombinant production of polypeptides of the invention. Such methods may involve the culture of host cells in an appropriate medium in conditions suitable for or conducive to expression of a polypeptide of the invention. The expressed recombinant polypeptide, which may optionally be secreted into the culture, may then be separated from the medium, host cells or culture medium by methods well known in the art (e.g. Deutscher, Ed, 1990, Methods in Enzymology, Vol 182, Guide to Protein Purification).

### Methods for producing plant cells and plants comprising constructs and vectors

Also described herein are plant cells which comprise a genetic construct described herein, and plant cells modified to alter expression of a polynucleotide or polypeptide described herein, or used in the methods of the invention. Plants comprising such cells are also provided.

Alteration of post-harvest storage characteristics may be altered in a plant through methods of the invention. Such methods may involve the transformation of plant cells and plants, with a construct designed to alter expression of a polynucleotide or polypeptide which modulates post-harvest storage characteristics in such plants. Such methods also include the transformation of plant cells and plants with a combination of the construct described herein and one or more other constructs designed to alter expression of one or more polynucleotides or polypeptides which modulate post-harvest storage characteristics in plants.

Methods for transforming plant cells, plants and portions thereof with polypeptides are described in Draper et al., 1988, Plant Genetic Transformation and Gene Expression. A Laboratory Manual. Blackwell Sci. Pub. Oxford, p. 365; Potrykus and Spangenburg, 1995, Gene Transfer to Plants. Springer-Verlag, Berlin.; and Gelvin et al., 1993, Plant Molecular Biol. Manual. Kluwer Acad. Pub. Dordrecht. A review of transgenic plants, including transformation techniques, is provided in Galun and Breiman, 1997, Transgenic Plants. Imperial College Press, London.

### Methods for genetic manipulation of plants

A number of plant transformation strategies are available (e.g. Birch, 1997, Ann Rev Plant Phys Plant Mol Biol, 48, 297, Hellens RP, et al (2000) Plant Mol Biol 42: 819-32, Hellens R et al Plant Meth 1: 13). For example, strategies may be designed to increase expression of a polynucleotide/polypeptide in a plant cell, organ and/or at a particular developmental stage where/when it is normally expressed or to ectopically express a polynucleotide/polypeptide in a cell, tissue, organ and/or at a particular developmental stage which/when it is not normally expressed. The expressed polynucleotide/polypeptide may be derived from the plant species to be transformed or may be derived from a different plant species.

Transformation strategies may be designed to reduce expression of a polynucleotide/polypeptide in a plant cell, tissue, organ or at a particular developmental stage which/when it is normally expressed. Such strategies are known as gene silencing strategies.

Genetic constructs for expression of genes in transgenic plants typically include promoters for driving the expression of one or more cloned polynucleotide, terminators and selectable marker sequences to detect presence of the genetic construct in the transformed plant.

The promoters suitable for use in the constructs described herein are functional in a cell, tissue or organ of a monocot or dicot plant and include cell-, tissue-and organ-specific promoters, cell cycle specific promoters, temporal promoters, inducible promoters, constitutive promoters that are active in most plant tissues, and recombinant promoters. Choice of promoter will depend upon the temporal and spatial expression of the cloned polynucleotide, so desired. The promoters may be those normally associated with a transgene of interest, or promoters which are derived from genes of other plants, viruses, and plant pathogenic bacteria and fungi. Those skilled in the art will, without undue experimentation, be able to select promoters that are suitable for use in modifying and modulating plant traits using genetic constructs comprising the polynucleotide sequences described herein. Examples of constitutive plant promoters include the CaMV 35S promoter, the nopaline synthase promoter and the octopine synthase promoter, and the Ubi 1 promoter from maize. Plant promoters which are active in specific tissues, respond to internal developmental signals or external abiotic or biotic stresses are described in the scientific literature. Exemplary promoters are described, e.g., in WO 02/00894.

Exemplary terminators that are commonly used in plant transformation genetic construct include, e.g., the cauliflower mosaic virus (CaMV) 35S terminator, the *Agrobacterium tumefaciens* nopaline synthase or octopine synthase terminators, the *Zea mays* zein gene terminator, the *Oryza sativa* ADP-glucose pyrophosphorylase terminator and the *Solanum tuberosum* PI-II terminator.

Selectable markers commonly used in plant transformation include the neomycin phophotransferase II gene (NPT II) which confers kanamycin resistance, the aadA gene, which confers spectinomycin and streptomycin resistance, the phosphinothricin acetyl transferase (*bar* gene) for Ignite (AgrEvo) and Basta (Hoechst) resistance, and the hygromycin phosphotransferase gene (hpt) for hygromycin resistance.

Use of genetic constructs comprising reporter genes (coding sequences which express an activity that is foreign to the host, usually an enzymatic activity and/or a visible signal (e.g., luciferase, GUS, GFP) which may be used for promoter expression analysis in plants and plant tissues are also contemplated. The reporter gene literature is reviewed in Herrera-Estrella et al., 1993, Nature 303, 209, and Schrott, 1995, In: Gene Transfer to Plants (Potrykus, T., Spangenberg. Eds) Springer Verlag. Berline, pp. 325-336.

Gene silencing strategies may be focused on the gene itself or regulatory elements which effect expression of the encoded polypeptide. "Regulatory elements" is used here in the widest possible sense and includes other genes which interact with the gene of interest.

Genetic constructs designed to decrease or silence the expression of a polynucleotide/polypeptide of the invention may include an antisense copy of a polynucleotide described herein. In such constructs the polynucleotide is placed in an antisense orientation with respect to the promoter and terminator.

An "antisense" polynucleotide is obtained by inverting a polynucleotide or a segment of the polynucleotide so that the transcript produced will be complementary to the mRNA transcript of the gene, e.g.,

| | |
|---|---|
| 5'GATCTA 3' (coding strand) | 3'CTAGAT 5' (antisense strand) |
| 3'CUAGAU 5' mRNA | 5'GAUCUCG 3' antisense RNA |

Genetic constructs designed for gene silencing may also include an inverted repeat. An 'inverted repeat' is a sequence that is repeated where the second half of the repeat is in the complementary strand, e.g.,
5'-GATCTA......... TAGATC-3'
3'-CTAGAT.........ATCTAG-5'

The transcript formed may undergo complementary base pairing to form a hairpin structure. Usually a spacer of at least 3-5 bp between the repeated region is required to allow hairpin formation. Constructs including such invented repeat sequences may be used in RNA interference (RNAi) and therefore can be referred to as RNAi constructs.

Another silencing approach involves the use of a small antisense RNA targeted to the transcript equivalent to an miRNA (Llave et al., 2002, Science 297, 2053). Use of such small antisense RNA corresponding to polynucleotide of the invention is expressly contemplated.

The term genetic construct as used herein also includes small antisense RNAs and other such polypeptides effecting gene silencing.

Transformation with an expression construct, as herein defined, may also result in gene silencing through a process known as sense suppression (e.g. Napoli et al., 1990, Plant Cell 2, 279; de Carvalho Niebel et al., 1995, Plant Cell, 7, 347). In some cases sense suppression may involve over-expression of the whole or a partial coding sequence but may also involve expression of non-coding region of the gene, such as an intron or a 5' or 3' untranslated region (UTR). Chimeric partial sense constructs can be used to coordinately silence multiple genes (Abbott et al., 2002, Plant Physiol. 128(3): 844-53; Jones et al., 1998, Planta 204: 499-505). The use of such sense suppression strategies to silence the expression of a polynucleotide of the invention is also contemplated.

The polynucleotide inserts in genetic constructs designed for gene silencing may correspond to coding sequence and/or non-coding sequence, such as promoter and/or intron and/or 5' or 3' UTR sequence, of the corresponding gene.

Other gene silencing strategies include dominant negative approaches and the use of ribozyme constructs (McIntyre, 1996, Transgenic Res, 5, 257).

Pre-transcriptional silencing may be brought about through mutation of the gene itself or its regulatory elements. Such mutations may include point mutations, frameshifts, insertions, deletions and substitutions.

The following are representative publications disclosing genetic transformation protocols that can be used to genetically transform the following plant species: Rice (Alam et al., 1999, Plant Cell Rep. 18, 572); apple (Yao et al., 1995, Plant Cell Reports 14, 407-412); maize (US Patent Serial Nos. 5, 177, 010 and 5, 981, 840); wheat (Ortiz et al., 1996, Plant Cell Rep. 15, 1996, 877); tomato (US Patent Serial No. 5, 159, 135); potato (Kumar et al., 1996 Plant J. 9,: 821); cassava (Li et al., 1996 Nat. Biotechnology 14, 736); lettuce (Michelmore et al., 1987, Plant Cell Rep. 6, 439); tobacco (Horsch et al., 1985, Science 227, 1229); cotton (US Patent Serial Nos. 5, 846, 797 and 5, 004, 863); grasses (US Patent Nos. 5, 187, 073 and 6. 020, 539); peppermint (Niu et al., 1998, Plant Cell Rep. 17, 165); citrus plants (Pena et al., 1995, Plant Sci.104, 183); caraway (Krens et al., 1997, Plant Cell Rep, 17, 39); banana (US Patent Serial No. 5, 792, 935); soybean (US Patent Nos. 5, 416, 011; 5, 569, 834 ; 5, 824, 877 ; 5, 563, 04455 and 5, 968, 830); pineapple (US Patent Serial No. 5, 952, 543); poplar (US Patent No. 4, 795, 855); monocots in general (US Patent Nos. 5, 591, 616 and 6, 037, 522); brassica (US Patent Nos. 5, 188, 958 ; 5, 463, 174 and 5, 750, 871); cereals (US Patent No. 6, 074, 877); pear (Matsuda et al., 2005, Plant Cell Rep. 24(1):45-51); Prunus (Ramesh et al., 2006 Plant Cell Rep. 25(8):821-8; Song and Sink 2005 Plant Cell Rep. 2006 ;25(2):117-23; Gonzalez Padilla et al., 2003 Plant Cell Rep.22(1):38-45); strawberry (Oosumi et al., 2006 Planta. 223(6):1219-30; Folta et al., 2006 Planta Apr 14; PMID: 16614818), rose (Li et al., 2003), Rubus (Graham et al., 1995 Methods Mol Biol. 1995;44:129-33), tomato (Dan et al., 2006, Plant Cell Reports V25:432-441), apple (Yao et al., 1995, Plant Cell Rep. 14, 407-412) and *Actinidia eriantha* (Wang et al., 2006, Plant Cell Rep. 25,5: 425-31). Transformation of other species is also contemplated by the invention. Suitable methods and protocols are available in the scientific literature.

Several further methods known in the art may be employed to alter expression of activity of a nucleotide and/or polypeptide described herein. Such methods include but are not limited to Tilling (Till et al., 2003, Methods Mol Biol, 2%, 205), so called "Deletagene" technology (Li et al., 2001, Plant Journal 27(3), 235) and the use of artificial transcription factors such as synthetic zinc finger transcription factors. (e.g. Jouvenot et al., 2003, Gene Therapy 10, 513). Additionally antibodies or fragments thereof, targeted to a particular polypeptide may also be expressed in plants to modulate the activity of that polypeptide (Jobling et al., 2003, Nat. Biotechnol., 21(1), 35). Transposon tagging approaches may also be applied. Additionally peptides interacting with a polypeptide described herein may be identified through technologies such as phase-display (Dyax Corporation). Such interacting peptides may be expressed in or applied to a plant to affect activity of a polypeptide of the invention. Use of each of the above approaches in alteration of expression of a nucleotide and/or polypeptide of the invention is specifically contemplated.

The terms "to alter expression of" and "altered expression" of a polynucleotide or polypeptide in the methods of the invention, are intended to encompass the situation where genomic DNA corresponding to a polynucleotide of the described herein is modified thus leading to altered expression of a polynucleotide or polypeptide of the invention. Modification of the genomic DNA may be through genetic transformation or other methods known in the art for inducing mutations. The "altered expression" can be related to an increase or decrease in the amount of messenger RNA and/or polypeptide produced and may also result in altered activity of a polypeptide due to alterations in the sequence of a polynucleotide and polypeptide produced.

### Methods of selecting plants

Methods are also provided for selecting plants with altered post-harvest storage characteristics. Such methods involve testing of plants for altered for the expression of a polynucleotide or polypeptide of the invention, or disclosed herein. Such methods may be applied at a young age or early developmental stage when the altered post-harvest storage characteristics may not necessarily be easily measurable.

The expression of a polynucleotide, such as a messenger RNA, is often used as an indicator of expression of a corresponding polypeptide. Exemplary methods for measuring the expression of a polynucleotide include but are not limited to Northern analysis, RT-PCR and dot-blot analysis (Sambrook et al., Molecular Cloning : A Laboratory Manual, 2nd Ed. Cold Spring Harbor Press, 1987). Polynucleotides or portions of the polynucleotides described herein are thus useful as probes or primers, as herein defined. The polynucleotides disclosed herein may be used as probes in hybridization experiments, or as primers in PCR based experiments, designed to identify such plants.

Alternatively antibodies may be raised against polypeptides described herein, or used in the methods described herein. Methods for raising and using antibodies are standard in the art (see for example: Antibodies, A Laboratory Manual, Harlow A Lane, Eds, Cold Spring Harbour Laboratory, 1998). Such antibodies may be used in methods to detect altered expression of polypeptides which modulate flower size in plants. Such methods may include ELISA (Kemeny, 1991, A Practical Guide to ELISA, NY Pergamon Press) and Western analysis (Towbin & Gordon, 1994, J Immunol Methods, 72, 313).

These approaches for analysis of polynucleotide or polypeptide expression and the selection of plants with altered post-harvest storage characteristics are useful in conventional breeding programs designed to produce varieties with altered post-harvest storage characteristics.

### Plants

The term "plant" is intended to include a whole plant, any part of a plant, a seed, a fruit, propagules and progeny of a plant.

The term 'propagule' means any part of a plant that may be used in reproduction or propagation, either sexual or asexual, including seeds and cuttings.

The plants described herein may be grown and either self-ed or crossed with a different plant strain and the resulting hybrids, with the desired phenotypic characteristics, may be identified. Two or more generations may be grown to ensure that the subject phenotypic characteristics are stably maintained and inherited.

### BRIEF DESCRIPTION OF THE DRAWINGS

The present invention will be better understood with reference to the accompanying drawings in which are described as follows:

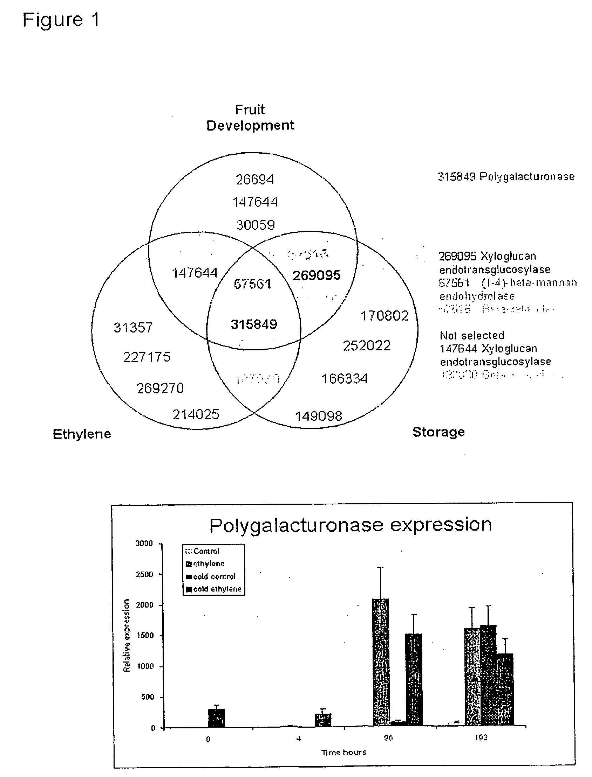

### Fig. 1: Selection of MdPG1 as a key gene in ripening apple fruit

A) The Venn diagram shows ESTs that significantly changed in expression using microarray analysis of RNA derived from three different treatments as described below: Fruit development: 8 time points from anthesis to ripe fruit, 0, 14, 25, 35, 60, 87, 132 and 146 days after full bloom. Ethylene induction: Ethylene knockout mutant apples induced to ripen with ethylene, and harvested 0,4,18,96 and 192 hours after ethylene induction. A 192 hour control (C) was also measured. Storage induction: Three different cultivars of apples were compared to each other before storage (Cultivar comparisons) and after storage at 0 °C for 4 weeks.
   The expression profiles of the 143 cell wall related genes were assessed in 3 unrelated microarray experiments that covered periods of physiological fruit softening: Microarray experiments were performed and analysed as described in Schaffer et al (2007).
B) Relative expression pattern of MdPG1 measured using qPCR
   RNA samples were extracted from ACC oxidase mutant apples to measure the effects of ethylene induced softening on PG expression with or without a cold storage treatment. Timepoints analysed by qPCR are shown in the schematic below cDNA was synthesized from 2 ug of total RNA in a total volume of 50 mL with Superscript III reverse transcriptase according to the manufacturer's instructions (Invitrogen). Controls with no Superscript III reverse transcriptase were used to assess for potential genomic DNA contamination. cDNA used for real-time RT-PCR was synthesized in triplicate and optical density was measured for each sample. qPCR reactions and normalization was performed as described in Schaffer et al (1998). Results show the strong induction of MdPG1 expression by ethylene treatment after 96 h and 192 h and by cold + ethylene treatment at 96 h and 192 h. MdPG1 expression is induced by cold treatment alone but more slowly (after 192 h).

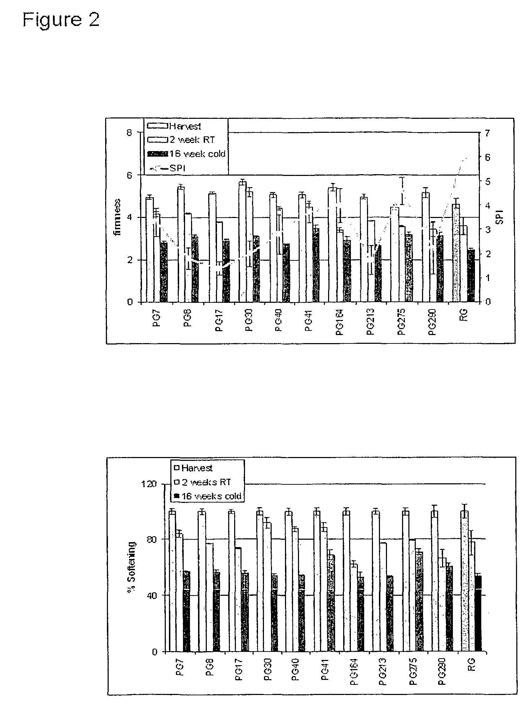

### Fig. 2: Firmness in transgenic PG knockout plants vs Royal Gala controls

Fruit firmness was measured destructively on individual fruit (n = 6-10 fruit per timepoint) at harvest, after 2 weeks storage at room temperature (∼20 °C) and after 16 weeks storage at 5 °C.

Firmness was measured using a puncture test according to standard industry practise (Blanpied et al., 1978). This involved the localised removal of skin from two opposing locations on the fruit equator, and recording the maximum force while driving a 7.9 mm cylindrical probe into the outer cortex to a constant depth (8 mm) at a fixed speed (4 mm/s). The puncture test and data capture was performed using a Stable Micro Systems TA-XT plus Texture Analyser (Hertog et al 2001;).
A) Fruit firmness in 10 knockout lines vs RG (Royal Gala) control.
B) Fruit firmness measurement expressed as a % of firmness at harvest.
SPI = starch pattern index measurements were made against commercial standards.

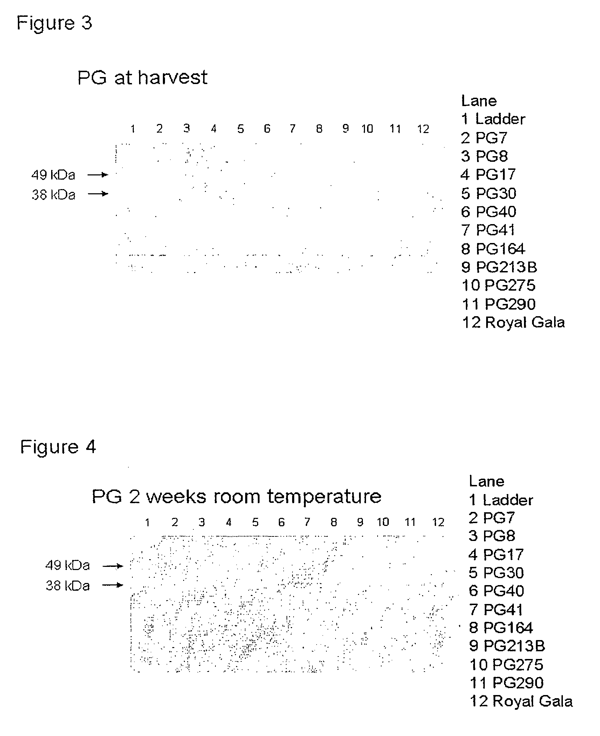

### Fig. 3: Western analysis of PG protein levels in transgenic Royal Gala apple fruit at harvest

Fruit tissues were ground to a powder with mortar and pestle under liquid nitrogen. Protein was extracted as described in Langenkapmper et al (1998). Proteins were separated on 12% (w/v) SDS-Tris-Tricine gels using a Mini-PROTEAN3 electrophoresis system (Bio-Rad, Hercules, CA, USA). Protein concentrations in each sample were measured using the QuBit Quantitation System (Invitrogen) and verified on gels by Coomassie staining. A polyclonal antibody raised to apple polygalacturonase was used to immunolocalise PG protein in transgenic and control Royal Gala apple plants.
Ladder = Precision Plus Protein Dual Colour Standards (Bio-Rad), sizing is in kDa.

### Fig. 4: Western analysis of PG protein levels in transgenic Royal Gala apple fruit after 2 weeks storage at room temperature (~20 °C)

Protein samples were extracted and analysed as described in Fig. 3.

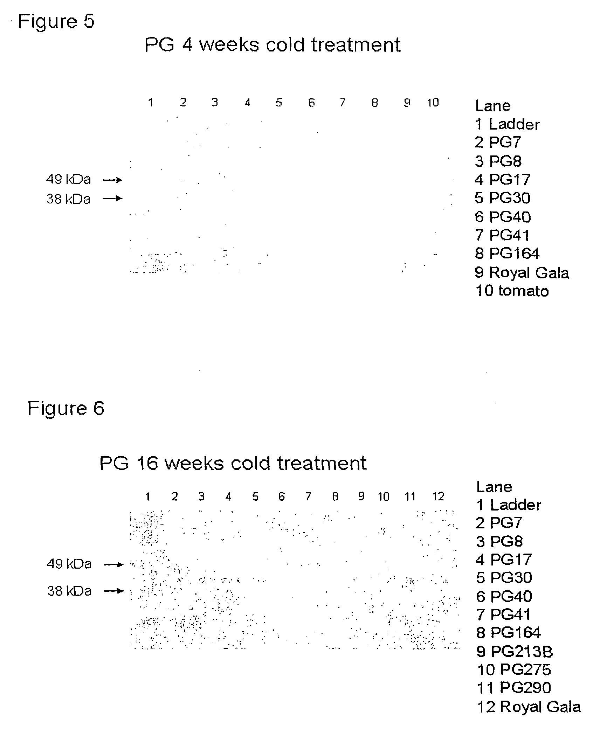

### Fig. 5: Western analysis of PG protein levels in transgenic Royal Gala apple fruit after 4 weeks storage at 5 °C

Protein samples were extracted and analysed as described in Fig. 3.

### Fig. 6: Western analysis of PG protein levels in transgenic Royal Gala apple fruit after 16 weeks storage at 5 °C

Protein samples were extracted and analysed as described in Fig. 3.

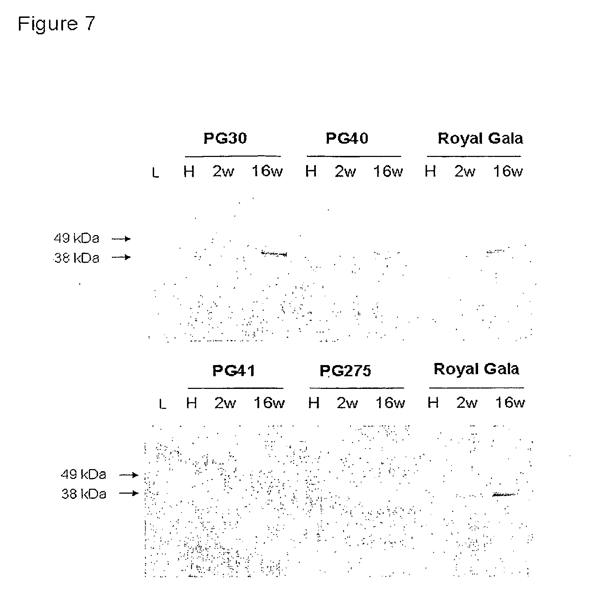

### Fig. 7: Western analysis of PG protein levels in six selected transgenic Royal Gala apple fruit

Protein samples were extracted and analysed as described in Fig. 3.
H = fruit at harvest
2w = fruit after 2 weeks storage at 5 °C
16w = fruit after 16 weeks storage at 5 °C
L = ladder, Precision Plus Protein Dual Colour Standards (Bio-Rad)

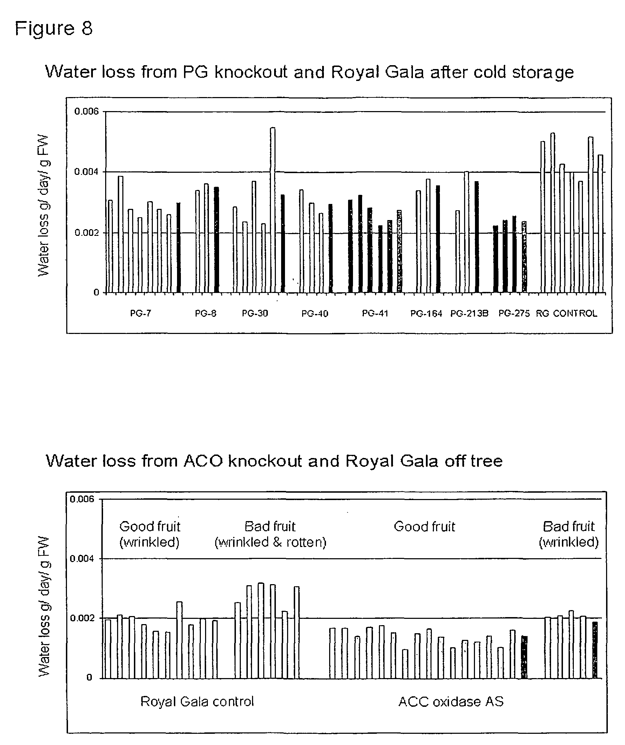

### Fig 8: Rate of water loss in transgenic vs control fruit

A - Rate of water loss from PG knockout and Royal Gala fruit after 16 weeks storage at 5°C followed by 5 weeks storage at room temperature. Water loss is expressed as grams of weight lost per day per g fresh weight of the fruit. Each bar represents an individual fruit.
Conclusion: Compared to control RG (Royal Gala) fruit, the two lines in which PG has been down-regulated most strongly (lines PG41 and PG275) show the lowest rate of water loss.

B - Comparison of water loss from Royal Gala control fruit and fruit in which the apple ACC oxidase gene has been knocked out (see Schaffer et al 1998 for description of this line). Nb this fruit was not cold stored suggesting that cold storing the apples may further accelerate water loss.

### Fig 9: Reduced wrinkling in transgenic Royal Gala fruit down-regulated for PG compared to Royal Gala control fruit

Fruit were stored for 16 weeks at 5 °C then transferred to room temperature for 5 weeks at room temperature. After this period transgenic fruit were substantially less wrinkled compared to controls which correlates with the reduced loss of water from these fruit.

### Fig. 10: Toluidine blue stained sections of apple from Royal Gala control and PG41 PG knockout line

Apple fruit cortex sections were fixed in glutaraldehyde and embedded in LR-white resin. Thin sections of 1 uM were stained with toluidine blue (0.1%). Sections were prepared from control Royal Gala fruit and the PG41 PG knockout line stored for 16 weeks at 5 °C. Arrows indicate points of pectin adhesion.
NB in control fruit the adhesion is reduced whilst in the PG41 lines the adhesion is maintained. This difference will have an effect on fruit softening and texture.

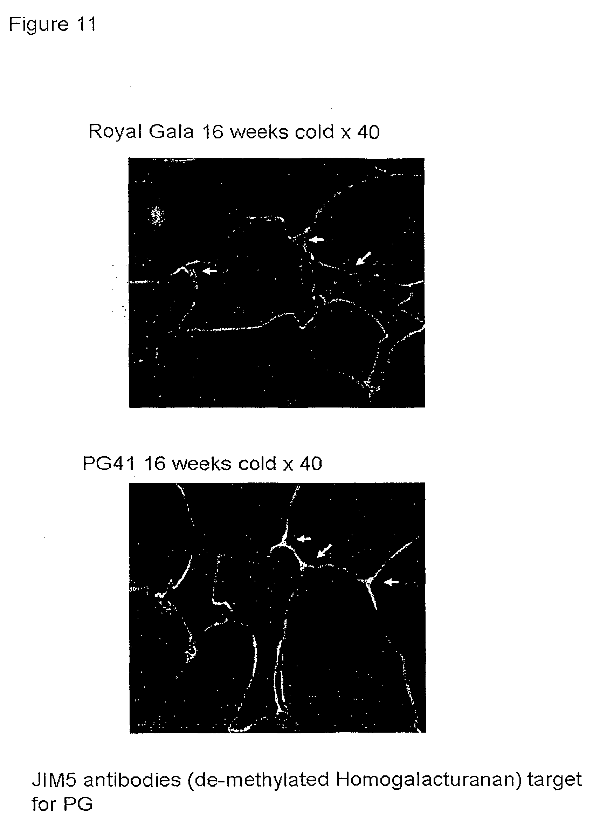

### Fig. 11: Immunolocalisation of non-esterified pectin from Royal Gala control and PG41 PG knockout line

Fixation of apple fruit tissues and immunolocalisation using JIM5 antibodies was performed as described in Atkinson et al. (2002). Sections were prepared from control Royal Gala fruit and the PG41 PG knockout line stored for 16 weeks at 5 °C. Magnification = x40. Arrows indicate points of JIM5 fluorescence.
NB in control fruit the fluorescence is reduced whilst in the PG41 lines the fluorescence is stronger. This result suggests that more demethylated homogalacturonan is present at the junction points between cells in PG41 fruit vs control fruit. This difference will have an effect on fruit softening and texture.

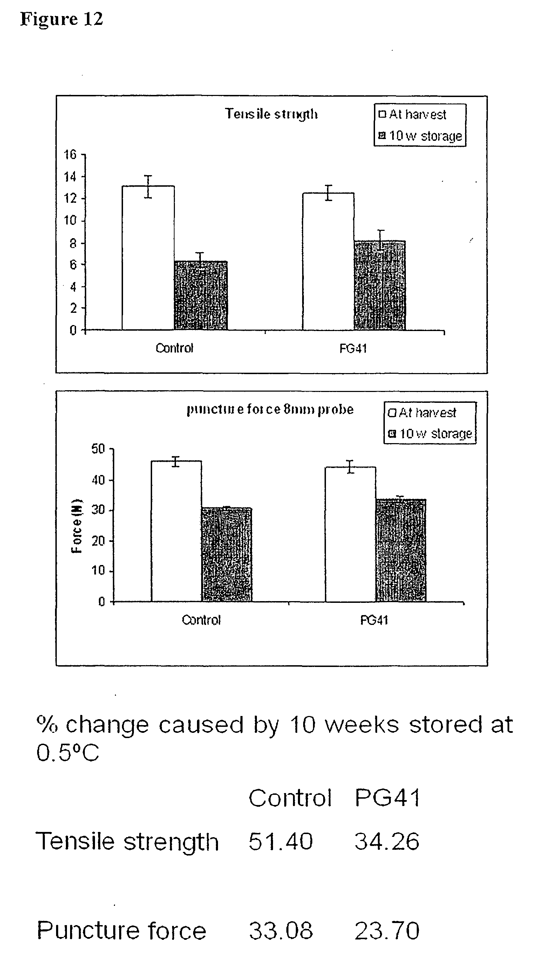

### Fig. 12: Tensile [pull apart] strength (upper panel) and flesh firmness measured with an 8mm probe (lower panel) for fruit of PG41 lines versus control (Royal Gala) lines at harvest (yellow bars) and after 10 weeks storage at 0.5°C (magenta bars).

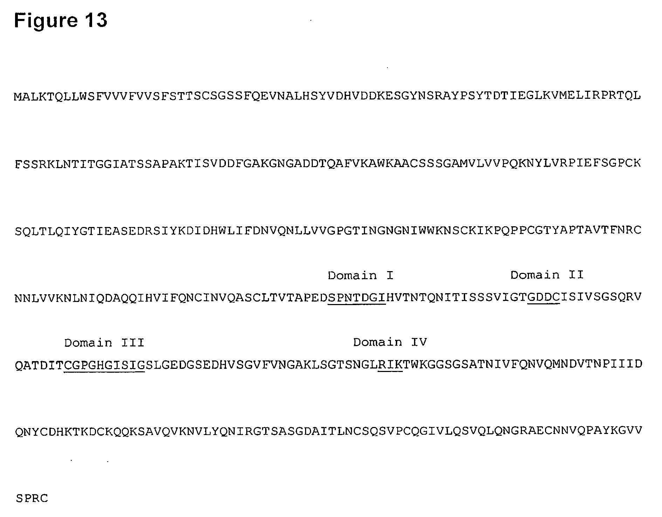

### Fig. 13: Amino acid sequence of MDPG1 highlighting conserved polygalacturonase domains

Figure 13 shows the position of four conserved domains (I to IV) that are present in the plant and fungal sequences (Torki et al. 2000 (incorporated herein by reference)). The carboxylate group in the three aspartic acids in NTD and DD structures (domains I and II, respectively) may be a component of the catalytic site and the histidine residue in domain III is thought to participate to the catalytic reaction. The well-conserved positively charged domain IV (RIK) constitutes a likely candidate for ionic interactions with carboxylate groups present in the substrate.

### EXAMPLES

The invention will now be illustrated with reference to the following non-limiting examples.

### EXAMPLE 1: Selection of MdPG1 as a candidate gene for altering post-harvest storage life in apple fruit

Three microarray experiments that measured apples that underwent ripening were analysed. These included an ethylene induced ripening series (Schaffer et al 2007), a fruit development series (Janssen et al 2008) and a cold storage treatment (manuscript in preparation). 290 cell wall related genes were identified by homology screening in the HortResearch Apple EST collection. Of these, 10 increased in expression late in fruit development, 9 increased in expression upon the addition of exogenous ethylene, and 10 increased in expression during 2 and a half months of cold storage. Of these genes, three were found to be in common to all treatments. Of the three, MdPG1 showed the greatest change in expression. Further analysis of this gene in transgenic apple lines down-regulated for the MdACO gene showed that MdPG1 is up-regulated in an ethylene dependent and cold dependent ripening manner (Figure 1).

Although reduction of expression of polygalacturonases (PGs) has been proposed as an approach to improving storage characteristics in fruit, success has been limited. This may be partly due to the large number of PGs that appear to be present in plants. For example Arabidopsis alone has been reported to contain approximately 52 different PG genes.

Transgenic plants have been used to study the role of endo-PGs in vivo. In tomato (*Lycopensicon esculentum*), down-regulation of the fruit-specific PG gene pTOM6 under the control of the constitutive cauliflower mosaic virus 35S promoter showed reduced depolymerization of pectin polymers in fruit (Smith et al., 1990). Overexpression of PG in the ripening inhibited mutant *rin* background restored PG activity and pectin degradation in fruit (Giovannoni et al., 1989). In both cases, only the fruit was affected by the transgene expression; therefore, the gene product isolated from tomato fruit appeared to have fruit specific PG activity. Further experiments where the pTOM6 gene was overexpressed in tobacco (*Nicotiana tabacum*; Osteryoung et al., 1990) showed that the tomato protein was properly processed and localized in the cell walls of leaves in tobacco. The enzyme showed activity when extracted from transgenic tobacco leaves and tested against tobacco cell wall extracts in vitro. However, no changes in leaf phenotype were observed, nor were there any alterations to the pectins in the tobacco cell walls in vivo. Expressing and given PG gene in plants theefore may give unpredictable results.

Apple (*Malus domestic* Borkh. cv Royal Gala) ripens very differently than tomato and many other fruits (Redgwell et al. 2008a; 2008b), because cell wall swelling is not one of the cell wall modifications occurring during apple ripening (Redgwell et al., 1997). There is minimal change in viscosity of cell walls, and minimal pectin solubilization or degradation during fruit ripening. This implies that any endo-PG isolated from ripening fruit of apple may have different characteristics to endo-PGs isolated from ripening tomato fruit. In a range of apple cultivars there is a suggestion that levels of polygalacturonase correlate with fruit firmness irrespective of ethylene production rate (Wakasa 2006).

### EXAMPLE 2: Production of plants with reduced expression of MdPG1

Ten transgenic 'Royal Gala' lines were created containing MdPG1 expressed in an antisense orientation driven by a strong constitutive promoter (35S promoter). The fruit-specific polygalacturonase cDNA clone MdPG1 (formerly GDPG1, Atkinson 1994), was cloned into pART7 as described previously (Atkinson et al. 2002). A clone with the PG gene in the antisense orientation was digested with *Not*I and cloned into the binary vector pART27. The binary was electroporated into *Agnobacterium tumefaciens* strain LBA4404. Transgenic apple 'Royal Gala' shoots were produced using the method of Yao et al. (1995) and maintained in a containment greenhouse under identical conditions (ambient light and temperature) to wild-type plants. Plants were transferred to chillers for 8-10 weeks each year to meet winter chilling requirements. Flowers were hand-pollinated each spring and fruit harvested in autumn when aroma volatiles could be detected.

### EXAMPLE 3: Fruit of plants produced by the methods of the invention show reduced softening during post-harvest storage

Five lines, of the 10 described in Example 2, showed less softening than the wild type control after two weeks at room temperature (Figure 2), and 2 lines (PG275 and PG41) showed significantly less softening after 16 weeks at 5°C storage. This correlated with previous experiments where apples from the line PG41 showed much reduced softening compared to the control apples. The decreased softening in this line has been shown for fruit collected over 3 growing seasons (Table 1).

Firmness was measured using a puncture test according to standard industry practise (Blanpied et al., 1978). This involved the localised removal of skin from two opposing locations on the fruit equator, and recording the maximum force while driving a 7.9 mm cylindrical probe into the outer cortex to a constant depth (8 mm) at a fixed speed (4 mm/s). The puncture test and data capture was performed using a Stable Micro Systems TA-XT plus Texture Analyser (Hertog et al 2001).

**Table 1 : Firmness in PG41 vs control apples over 3 years**

| | **2005** | | **2007** | | **2008** | |
|---|---|---|---|---|---|---|
| | **Harvest** | **Storage 30 wks, 5 °C** | **Harvest** | **Storage 32 wks, 5 °C** | **Harvest** | **Storage 16 wks, 5 °C** |
| **Probe (mm)** | **11** firmness | **11** firmness | **11** firmness | **11** firmness | **8.5** firmness | **8.5** firmness |
| PGA41 | no data | 6.18 | 9.25 | 7.0 | 5.06 | 3.50 |
| control | no data | 2.83 | 4.7 | 4.0 | 4.64 | 2.49 |

Firmer fruit is a desirable characteristic as sensory/consumer trials show that consumers prefer firmer fruit.

### EXAMPLE 4: Levels of MdPG1 protein correlate with rate of softening

The mature ORF of MdPG1 was amplified by PCR using primers RA136 5'-ACGGGATCCG CTCCGGCCAA AACCATTAGC-3' and RA137 5'-ATAGTTTAGC GGCCGCTTAA CATCTAGGGG AGACAAC-3'. The insert was excised with *Bam*HI and *Not*I (underlined in the primers) and ligated into corresponding sites of the pET-30a(+) vector (Novagen, Madison, WI, USA). pETMdPG1 was transformed into BL21 cells containing the pLysS plasmid and recombinant His-tagged protein purified by Ni-affinity chromatography under denaturing conditions (Schröder *et al.* 1998). Purified recombinant forms of MdPG1 protein cut from a polyacrylamide gel was used to raise a polyclonal antibody in rabbits.

Levels of MdPG1 protein were measured on western blots using polyclonal antibodies raised to the mature MdPG1 protein. For each transgenic line, described in Example 3 above, protein was extracted from apples at harvest, after two weeks room temperature storage and after 16 weeks cold storage. At harvest no MdPG1 was detected in any of the apples except line PG290 (Figure 3), After 2 weeks storage at room temperature (RT) it was found that both the 'Royal Gala' lines and PG290 showed a significant level of PG. Lines PG7, PG8, PG17, and PG164 had a detectable level of PG (Figure 4). After 4 weeks of cold storage lines PG7, PG8, PG30 and PG164 had a detectable level of PG (lines 213B, PG275 and PG290 were not assayed) (Figure 5). At 16 weeks storage lines except PG41 and PG275 showed significant levels of PG (Figure 6). Comparison across time points there was a strong correlation of levels of PG and rate of softening. Lines PG30 and PG40 showed little softening at 2 weeks RT and showed very low levels of PG at this time point. PG41 showed no detectable PG and PG275 showed very low levels of PG both of which were the firmest apples after 16 weeks storage (Figure 7).

### EXAMPLE 5: Fruit of plants produced by the methods of the invention show reduced water loss during post-harvest storage

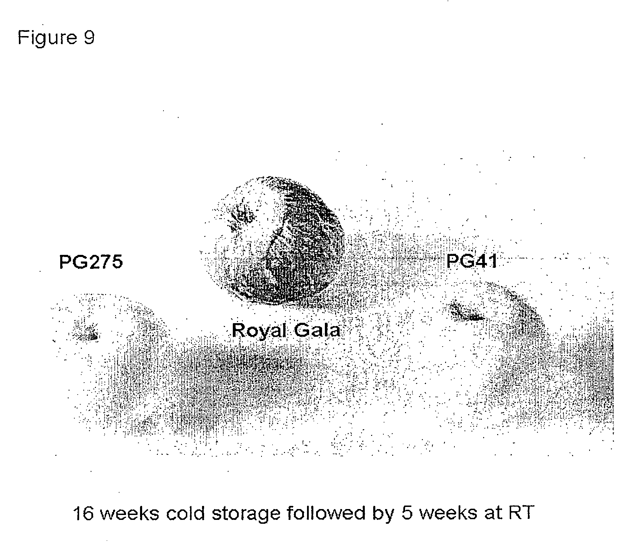

Apples from 8 independent transformant lines, along with the control 'Royal Gala' apples were left at room temperature for 1 month following a 16 week storage period at 4 degrees, and weighed every two weeks. It was found that the lines PG41 and PG275 showed a lower rate of water loss (0.00273 and 0.00237 g/day/g FW respectively) compared to the untransformed control (0.0046 g/day/g FW). The other transgenic lines showed a range between 0.00299 and 0.00317 g/day/g FW) (Figure 8). These numbers were much larger than those found in a separate water loss experiment with a non-ripening mutant ACO antisense apples and 'Royal Gala' lines that had not been cold stored (Figure 8), suggesting that cold storing the apples may further accelerate water loss. Apples from the PG41 and PG275 lines also showed less shrivelling compared at 5 weeks RT after transfer from 16 weeks cold storage (Figure 9).

### EXAMPLE 6: Fruit of plants produced by the methods of the invention show increased juiciness during post-harvest storage

### Microscopic analysis of PG41 lines and untransformed controls

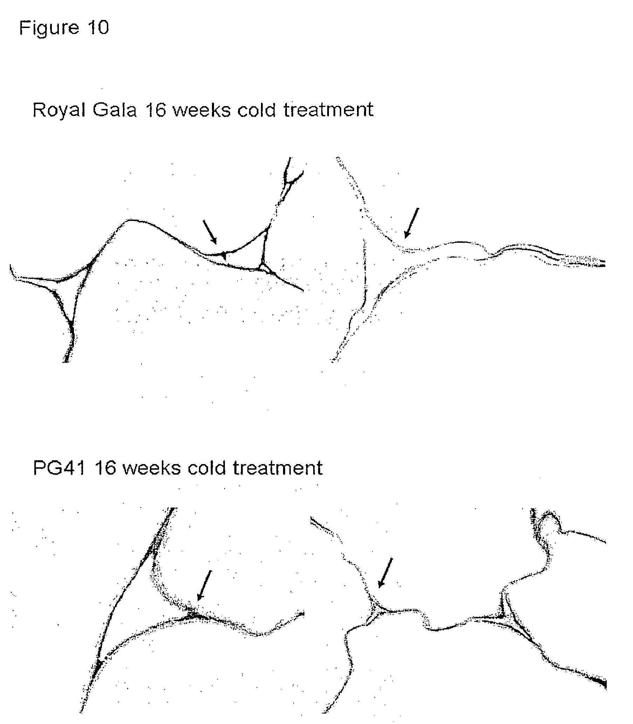

Sectioning cells from Royal Gala apples following a 16 week cold storage period revealed that the cell-to-cell adhesion was significantly weakened (with presumably pectin junctions between cells showing clear regions that have pulled apart Figure 10A). Sections of cells in the PG41 lines show no pulling apart (Figure 10B). Additionally antibody staining of the PG41 lines showed a maintainance of the demethylated homogalacturans in cell corners (Figure 11) compared to the 'Royal Gala' control, that are targeted by PG (identified using a JIM5 antibody). This suggests that decreasing the level of PG reduces cells breaking between the cell boundaries rather than across the cells. It has been proposed that that the difference between juicy apples and mealy apples is due to the way that the cells are disrupted during a bite action. Hallett et al have shown that juicyness is not a measure of water content rather as mealy and juicy apples contain the same amount of water. It has been suggested that juicy apples break across cells releasing the juice while mealy apples break between cells giving a much dryer mouth feel. The loss of cell to cell adhesion in the control lines suggest that the apples would have a more mealy texture, and the PG41 apples would be more juicy (this cannot be confirmed due to restrictions on eating transgenic apples in this country). From these results it is anticipated that the PG knockout apples would also be crisper and crunchier than the 'Royal gala' controls. When cutting the apples after storage they appeared to maintain their crispness.

### EXAMPLE 7: Fruit of plants produced by the methods of the invention show altered wax composition

PG antisense lines PG17 and PG275 both had a waxy feel compared to the Royal Gala control providing evidence that altered expression in the method of the invention can result in altered wax production.

### EXAMPLE 8: Fruit of plants produced by the methods of the invention show reduced post-harvest storage rots/infections

Control fruit were subject to infection by postharvest pathogens after long term storage at 5°C. In contrast PG41 lines rarely showed infection. This effect may be due to a reduction in microcracks on the surface of the fruit which provide an entry point for pathogen invasion.

### Fruit storage at 5°C

Commercial fruit storage is carried out at 1°C in controlled/mofified atmosphere conditions. PG41 apples in this study were stored at less than optimal conditions and still maintained fruit quality. This may allow fruit to stored at slightly higher temperatures thereby reducing costs.

### EXAMPLE 9: Fruit of plants produced by the methods of the invention show mostly normal ripening attributes

### PG41 apples show normal ripening attributes

To assess whether any other ripening attribute was altered in the PG41 mutant that may contribute to the phenotype, internal ethylenes, starch pattern index (SPI) and soluble solids content (SSC) were measured at harvest, and ethylene was measured 16 weeks after cold storage. From the SPI the Royal Gala apples appeared to be slightly more mature than the PG knock out lines at harvest, but after 16 weeks cold storage the PG41 lines and Royal Gala controls were producing similar amounts of ethylene (Figure 12), suggesting that the reduced softening is not due to decreased levels of ethylene.

### EXAMPLE 10: Identification of variants of the MdPG1

The MdPG1 sequence was used to identify orthologous PG genes from HortResearch proprietary sequence databases.

Two variant sequences were identified as summarised in the table below.

| **Polygalacturonase** | **Malus species** | **Polynucleotide SEQ ID NO:** | **Polypeptide SEQ ID NO:** |
|---|---|---|---|
| MsPG1 | *sieboldii* | 6 | 2 |
| MsPG2 | *sieboldii* | 7 | 3 |

The table below shows the % identity between the MdPG1 and variant polypeptide sequences.

| | MdPG1 | MsPG1 | MsPG2 |
|---|---|---|---|
| MdPG1 | 100% | 92.3 | 95.2 |
| MsPG1 | | 100% | No overlap |
| MsPG2 | | | 100% |

The function of these variants can be confirmed using the methods described in the examples above.

### EXAMPLE 11: Fruit of plants produced by the methods of the invention show increased tensile strength and firmness in commercial storage conditions

30 fruit from the PG41 lines which had no detectable fruit ripening endopolygalacturonase1 were harvested along with 30 untransformed Royal Gala (RG) controls. 15 fruit had 1 mm skin removed in 4 quadrants in the equatorial region of the apple and were measured at harvest from each line for puncture firmness was measured (with 4 different probe sizes) (Table 1a) using a TA.XT texture analyzer (Stable Micro Systems, Ltd, UK) as described in Johnston et al (2009). Cores from the cortex tissue were taken and tensile strength of these were measured using the TA.XT texture analyzer

15 apples from each line were stored at 0.5°C for 10 weeks under commercial storage conditions. Following this time apples were tested again for tensile strength and flesh puncture firmness. Additionally these samples were also assessed for amount of juice released by a commercial juicer to assess levels of juiciness.

**Table 1. Results of tensile strength and firmness**

| | | Absolute values | | % change during storage | |
|---|---|---|---|---|---|
| Data | Storage time (wks) | Control | PG41 | Control | PG41 |
| Average of Tens Max Force (N) | 0 | 13.1±1.0 | 12.5±0.6 | 51.40 | 34.26 |
| | 10 | 6.3±0.7 | 8.2±1.0 | | |
| Average of Force 11 mm | 0 | 84.4±2.2 | 78.6±3.3 | 30.94 | 16.91 |
| Probe (N) | 10 | 58.3±1.4 | 65.3±2.4 | | |
| Average of Force 8mm | 0 | 45.8±1.5 | 44.3±2.0 | 33.08 | 23.70 |
| Probe (N) | 10 | 30.6±0.6 | 33.8±1.0 | | |
| Average of Force 5mm | 0 | 18.7±0.6 | 18.9±0.8 | 28.37 | 22.73 |
| Probe (N) | 10 | 13.4±0.3 | 14.6±0.5 | | |
| Average of Force 2mm | 0 | 4.1±0.2 | 4.2±0.2 | 34.97 | 28.20 |
| Probe (N) | 10 | 2.7±0 | 3.0±0 | | |

There were no clear differences between the control RG lines and the PG 41 lines at harvest. But after 10 weeks storage there was a significant increase in both tensile strength and firmness. The 7 N increase (65.3 N - 58.3) in firmness of PG41 apples relative to RG control apples measured with the 11 mm probe following storage is larger than the minimum 6 N difference that a trained sensory panel can detect (Harker et al 2002) strongly indicating that in a sensory trial the PG41 fruit would be scored as better textured than the RG control after storage (Figure 12). When the original firmness of the PG41 fruit is taken into account then there is only a 16% loss of firmness compared to a 31 % loss of firmness in the control fruit.

### REFERENCES

Atkinson, R. G. 1994. A cDNA clone for endopolygalacturonase gene from apple. Plant Physiol. 195, 1437-1438.
Atkinson, R. G., Schröder, R., Hallett, I. C., Cohen, D., MacRae, E. A. 2002. Overexpression of polygalacturonase in transgenic apple trees leads to a range of novel phenotypes involving changes in cell adhesion. Plant Physiol. 129, 122-133.
Ayub, R., Guis, M., Ben Amor, M., Gillot, L., Roustan, J. P., Latche, A., Bouzayen, M., Pech, J. C. 1996. Expression of ACC oxidase antisense gene inhibits ripening of cantaloupe melon fruits. Nat. Biotechnol. 14, 862-6.
Blanpied GD, Bramlage WJ, Dewey DH, LaBelle RL, Massey LM, Mattus GE, Stiles WC, Watada AE. 1978. A standardized method for collecting apple pressure test data. New York's Food and Life Sciences Bulletin, 74 (August), 3-8.
Brummell, D. A., Harpster, M. H., Civello, P. M., Palys, J. M., Bennett, A. B., Dunsmuir, P. 1999. Modification of expansin protein abundance in tomato fruit alters softening and cell wall polymer metabolism during ripening. Plant Cell 11, 2203-16.
Cantu, D., Vicente, A. R., Greve, L. C., Dewey, F. M., Bennett, A. B., Labavitch, J. M., Powell, A. L. 2008. The intersection between cell wall disassembly, ripening, and fruit susceptibility to Botrytis cinerea. Proc. Natl. Acad. Sci. U. S. A. 105, 859-64.
Carey, A. T., Smith, D. L., Harrison, E., Bird, C. R., Gross, K. C., Seymour, G. B., Tucker, G. A. 2001. Down-regulation of a ripening-related beta-galactosidase gene (TBG1) in transgenic tomato fruits. J. Exp. Bot. 52, 663-668.
Carrington, C., Greve, L. C., Labavitch, J. M. 1993. Cell Wall Metabolism in Ripening Fruit (VI. Effect of the Antisense Polygalacturonase Gene on Cell Wall Changes Accompanying Ripening in Transgenic Tomatoes). Plant Physiol. 103, 429-434.
Giovannoni, J. J., DellaPenna, D., Bennett, A. B., Fischer, R. L. 1989. Expression of a chimeric polygalacturonase gene in transgenic rin (ripening inhibitor) tomato fruit results in polyuronide degradation but not fruit softening. Plant Cell 1, 53-63.
Hellens, R. P., Allan, A. C., Friel, E. N., Bolitho, K., Grafton, K., Templeton, M. D., Karunairetnam, S., Gleave, A. P. and Laing, W. A. (2005) Transient expression vectors for functional genomics, quantification of promoter activity and RNA silencing in plants. Plant Methods 1, 13
Hamilton, A. J., Lycett, G. W., Grierson, D. 1990. Antisense Gene That Inhibits Synthesis of the Hormone Ethylene in Transgenic Plants. Nature 346,284-287.
Harker, F.R., Maindonald, J., Murray, S. H., Gunson, F. A., Hallett, I.C. and Walker, S. B. 2002. Sensory interpretation of instrumental measurements 1: texture of apple fruit. Postharvest Biology and Technology, 24, 225-239.
Hertog MLATM, Nicholson SE, Banks N. 2001. The effect of modified atmospheres on the rate of firmness changes in 'Braeburn' apples. Postharvest Biology and Technology, 23, 175-184.
Janssen, B. J., Thodey, K., Schaffer, R. J., Alba, R., Balakrishnan, L., Bishop, R., Bowen, J. H., Crowhurst, R. N., Gleave, A. P., Ledger, S., McArtney, S., Pichler, F. B., Snowden, K. C., Ward, S. 2008. Global gene expression analysis of apple fruit development from the floral bud to ripe fruit. BMC Plant Biol 8, 16.
Johnston JW, Gunaseelan K, Pidakala P, Wang M, Schaffer RJ. (2009) Coordination of early and late ripening events in apples is regulated through differential sensitivities to ethylene. J. Exp. Bot 60, 2689-2699
Langenkamper, G., McHale, R., Gardner, R. C., MacRae, E. 1998. Sucrose-phosphate synthase steady-state mRNA increases in ripening kiwifruit. Plant Mol. Biol. 36, 857-69.
Langley, K. R., Martin, A., Stenning, R., Murray, A. J., Hobson, G. E., Schuch, W. W., Bird, C. R. 1994. Mechanical and Optical Assessment of the Ripening of Tomato Fruit with Reduced Polygalacturonase Activity. J. Sci. Food Agric. 66, 547-554.
Oeller, P. W., Lu, M. W., Taylor, L. P., Pike, D. A., Theologis, A. 1991. Reversible inhibition of tomato fruit senescence by antisense RNA. Science 254, 437-9.
Osteryoung, K. W., Toenjes, K., Hall, B., Winkler, V., Bennett, A. B. 1990. Analysis of tomato polygalacturonase expression in transgenic tobacco. Plant Cell 2, 1239-48.
Powell, A. L., Kalamaki, M. S., Kurien, P. A., Gurrieri, S., Bennett, A. B. 2003. Simultaneous transgenic suppression of LePG and LeExp1 influences fruit texture and juice viscosity in a fresh market tomato variety. J. Agric. Food Chem. 51, 7450-5.
Schröder, R., Atkinson, R. G., Langenkamper, G., Redgwell, R. J. 1998. Biochemical and molecular characterisation of xyloglucan endotransglycosylase from ripe kiwifruit. Planta 204, 242-251.
Redgwell, R. J., Curti, D., Gehin-Delval, C. 2008a. Physicochemical properties of cell wall materials from apple, kiwifruit and tomato. European Food Research and Technology 227, 607-618.
Redgwell, R. J., Curti, D., Gehin-Delval, C. 2008b. Role of pectic polysaccharides in structural integrity of apple cell wall material. European Food Research and Technology 227, 1025-1033.
Redgwell, R. J., Fischer, M., Kendal, E., MacRae, E. A. 1997. Galactose loss and fruit ripening: high-molecular-weight arabinogalactans in the pectic polysaccharides of fruit cell walls. Planta 203, 174-181.
Saladié, M., Rose, J. K. C., Cosgrove, D. J., Catalá, C. 2006. Characterization of a new xyloglucan endotransglucosylase/hydrolase (XTH) from ripening tomato fruit and implications for the diverse modes of enzymic action. Plant J. 47, 282-295.
Santiago-Domenech, N., Jimenez-Bemudez, S., Matas, A. J., Rose, J. K., Munoz-Blanco, J., Mercado, J. A., Quesada, M. A. 2008. Antisense inhibition of a pectate lyase gene supports a role for pectin depolymerization in strawberry fruit softening. J. Exp. Bot. 59, 2769-79.
Schaffer, R. J., Friel, E. N., Souleyre, E. J., Bolitho, K., Thodey, K., Ledger, S., Bowen, J. H., Ma, J. H., Nain, B., Cohen, D., Gleave, A. P., Crowhurst, R. N., Janssen, B. J., Yao, J. L., Newcomb, R. D. 2007. A genomics approach reveals that aroma production in apple is controlled by ethylene predominantly at the final step in each biosynthetic pathway. Plant Physiol. 144, 1899-912.
Schuch, W., Kanczler, J., Robertson, D., Hobson, G., Tucker, G., Grierson, D., Bright, S., Bird, C. 1991. Fruit-Quality Characteristics of Transgenic Tomato Fruit with Altered Polygalacturonase Activity. HortScience 26, 1517-1520.
Smith, C. J., Watson, C. F., Bird, C. R., Ray, J., Schuch, W., Grierson, D. 1990. Expression of a truncated tomato polygalacturonase gene inhibits expression of the endogenous gene in transgenic plants. Mol. Gen. Genet. 224, 477-81.
Torki, M., Mandaron, P., Mache, R., and Falconet, D. 2000. Characterization of a ubiquitous expressed gene family encoding polygalacturonase in Arabidopsis thaliana. Gene. 2000 Jan 25;242(1-2):427-36.
Wakasa, Y., Kudo, H., Ishikawa, R., Akada, S., Senda, M., Niizeki, M., Harada, T. 2006. Low expression of an endopolygalacturonase gene in apple fruit with long-term storage potential. Postharvest Biol. Technol. 39, 193-198.
Yao, J. L., Cohen, D., Atkinson, R., Richardson, K., Morris, B. 1995. Regeneration of transgenic plants from the commercial apple cultivar Royal Gala. Plant Cell Reports 14, 407-412.
van Rensburg, P., van Zyl, W.H., Pretorius I.S. 1994. Expression of the Butyrivibrio fibrisolvens endo-beta-1,4-glucanase gene together with the Erwinia pectate lyase and polygalacturonase genes in Saccharomyces cerevisiae. Curr Genet. 27 (1):17-22.

### SUMMARY OF SEQUENCES

| SEQ ID NO: | Type | Species | Reference |
|---|---|---|---|
| 1 | polypeptide | *Malus x domesticα* | MdPG1, polygalacturonase, Genbank accession number L27743 |
| 2 | polypeptide | *Malus sieboldii* | MsPG1, polygalacturonase |
| 3 | polypeptide | *Malus sieboldii* | MsPG2, polygalacturonase |
| 4 | polynucleotide | *Mains x domestica*, | MdPG1, polygalacturonase promoter sequence and part of first exon. Genbank accession number AF031233) |
| 5 | polynucleotide | *Malus x domestica,* | MdPG1, polygalacturonase, cDNA |
| 6 | polynucleotide | *Malus sieboldii* | MsPG1, polygalacturonase, cDNA |
| 7 | polynucleotide | *Malus sieboldii* | MsPG2, polygalacturonase, cDNA |

## Claims

1. A method for increasing post-harvest storage life and firmness of fruit of a plant from a Rosaceae species during, or after, post-harvest storage, relative to the fruit of a control plant under the same conditions, the method comprising reducing the expression or activity in the plant, of a polypeptide with the amino acid sequence of SEQ ID NO: 1, or a variant of the polypeptide with at least 70% identity to the amino acid sequence of SEQ ID NO: 1 over the entire length of SEQ ID NO: 1, wherein the method comprises the step of introducing a polynucleotide into a plant cell, or plant, to effect reducing the expression of the polypeptide or variant, wherein the polynucleotide comprises at least one of:
i) a sequence with at least 70% identity to part of an endogenous gene, or nucleic acid, that encodes the polypeptide or variant thereof, and
ii) a sequence that hybridizes under stringent conditions to part of an endogenous gene, or nucleic acid, encoding the polypeptide or variant thereof, wherein the variant has polygalacturonase activity.

2. The method of claim 1 in which the fruit also have at least one of:
a) reduced water loss,
b) reduced cell separation,
c) increased juiciness,
d) increased crispiness,
e) increased waxiness, and
f) reduced susceptibility to necrophytic pathogens,
during, or after, post-harvest storage, relative to the fruit of a control plant under the same conditions.

3. The method of any one of claims 1 and 2 in which the polynucleotide is introduced into the plant as part of a genetic construct.

4. The method of any one of claims 1 to 3 in which the polynucleotide is introduced into the plant as part of an expression construct comprising a promoter operably linked to the polynucleotide.

5. The method of claim 4 in which the polynucleotide in an antisense orientation relative to the promoter.

6. The method of any one of claims 1 to 5, wherein the plant is from the *Malus* genus.

7. The method of claim 6, wherein the plant is *Malus domestica.*

## Patentansprüche

1. Verfahren zur Erhöhung der Lagerfähigkeit nach der Ernte und der Festigkeit der Frucht einer Pflanze einer Rosaceae-Spezies während oder nach der Lagerung nach der Ernte in Bezug auf das Obst einer Vergleichspflanze unter den gleichen Bedingungen, wobei das Verfahren das Reduzieren der in der Pflanze stattfindenden Expression oder Aktivität eines Polypeptids mit der Aminosäuresequenz von Seq.-ID Nr. 1 oder einer Variante des Polypeptids mit zumindest 70 % Identität mit der Aminosäuresequenz von Seq.-ID Nr. 1 über die gesamte Länge von Seq.-ID Nr. 1 umfasst, worin das Verfahren den Schritt des Einführens eines Polynucleotids in eine Pflanzenzelle oder Pflanze umfasst, um das Reduzieren der Expression des Polypeptids oder der Variante zu bewirken, worin das Polynucleotid zumindest eines der Folgenden umfasst:
i) eine Sequenz mit zumindest 70 % Identität mit einem Teil eines endogenen Gens oder einer Nucleinsäure, das/die für das Polypeptid oder dessen Variante kodiert, und
ii) eine Sequenz, die unter stringenten Bedingungen an einen Teil eines endogenen Gens oder eine Nucleinsäure hybridisiert, das/die für das Polypeptid oder dessen Variante kodiert,
worin die Variante Polygalacturonase-Aktivität aufweist.

2. Verfahren nach Anspruch 1, worin die Frucht auch zumindest eines der Folgenden aufweist:
a) reduzierten Wasserverlust,
b) reduzierte Zellteilung,
c) erhöhte Saftigkeit,
d) erhöhte Knackigkeit,
e) erhöhte Wachsartigkeit,
f) reduzierte Anfälligkeit gegenüber nekrophytischen Pathogenen
während oder nach der Lagerung nach der Ernte in Bezug auf die Frucht einer Vergleichspflanze unter den gleichen Bedingungen.

3. Verfahren nach einem der Ansprüche 1 und 2, in dem das Polynucleotid als Teil eines genetischen Konstrukts in die Pflanze eingeführt wird.

4. Verfahren nach einem der Ansprüche 1 bis 3, worin das Polynucleotid als Teil eines Expressionskonstrukts in die Pflanze eingeführt wird, das einen mit dem Polynucleotid operabel verbundenen Promotor umfasst.

5. Verfahren nach Anspruch 4, worin das Polynucleotid in Bezug auf den Promotor in Antisense-Ausrichtung vorliegt.

6. Verfahren nach einem der Ansprüche 1 bis 5, worin die Pflanze aus der Gattung *Malus* stammt.

7. Verfahren nach Anspruch 6, worin die Pflanze *Malus* domestica ist.

## Revendications

1. Procédé pour augmenter la durée de stockage après la récolte et la fermeté de fruits d'une plante de la famille des Rosacées, pendant ou après le stockage après la récolte, par rapport aux fruits d'une plante de comparaison sous les mêmes conditions, le procédé comprenant réduire l'expression, ou l'activité dans une plante, d'un polypeptide avec la séquence d'acides aminés de SEQ ID NO : 1, ou d'une variante du polypeptide avec au moins 70 % d'identité avec la séquence d'acides aminés de SEQ ID NO : 1 sur la longueur totale de SEQ ID NO : 1, le procédé comprenant l'étape d'introduction d'un polynucléotide dans une cellule de plante, ou d'une plante, pour effectuer une réduction de l'expression du polypeptide ou de la variante, le polynucléotide comprenant au moins un parme :
i) une séquence avec au moins 70 % d'identité avec une partie d'un gène endogène, ou d'un acide nucléique, qui encode le polypeptide ou la variante de celui-ci, et
ii) une séquence qui hybride, sous des conditions rigoureuses, une partie d'un gène endogène, ou d'un acide nucléique, qui encode le polypeptide ou la variante de celui-ci, la variante ayant une activité de polygalacturonase.

2. Procédé selon la revendication 1, **caractérisé en ce que** les fruits présentent aussi au moins une caractéristique parme :
a) perte réduite d'eau,
b) séparation réduite de cellules,
c) jutosité augmentée,
d) apparence croustillante augmentée,
e) effet de cire augmenté, et
f) sensibilité réduite aux pathogènes nécrophytiques,
pendant ou après le stockage après la récolte, par rapport aux fruits d'une plante de comparaison sous les mêmes conditions.

3. Procédé selon l'une quelconque des revendication 1 et 2, **caractérisé en ce que** le polynucléotide est introduit dans la plante comme une partie d'une construction génétique.

4. Procédé selon l'une quelconque des revendications 1 à 3, **caractérisé en ce que** le polynucléotide est introduit dans la plante comme une partie d'une construction d'expression comprenant un promoteur lié en fonctionnement au polynucléotide.

5. Procédé selon la revendication 4, **caractérisé en ce que** le polynucléotide est orienté en sens inverse par rapport au promoteur.

6. Procédé selon l'une quelconque des revendications 1 à 5, **caractérisé en ce que** la plante est de type *Malus.*

7. Procédé selon la revendication 6, **caractérisé en ce que** la plante est *Malus domestica.*
